(19) Europäisches Patentamt
European Patent Office
Office européen des brevets

(11) EP 4 730 342 A1

(12) EUROPEAN PATENT APPLICATION

(43) Date of publication:
22.04.2026 Bulletin 2026/17

(51) International Patent Classification (IPC):
G16B 40/20 (2019.01)

(21) Application number: 24306746.9

(52) Cooperative Patent Classification (CPC):
G16B 40/20; G06N 20/10

(22) Date of filing: 18.10.2024

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Sanofi
75017 Paris (FR)

(72) Inventors:
• DE GROEVE, Manu
  9052 Zwijnaarde (BE)
• AMENGUAL-RIGO, José Antonio
  65929 Frankfurt (DE)

(74) Representative: Trichard, Louis
Venner Shipley LLP
200 Aldersgate
London EC1A 4HD (GB)

(54) **METHOD, SYSTEM AND APPARATUS FOR ESTIMATING TITER FOR IMMUNOGLOBULIN SINGLE VARIABLE DOMAIN MOLECULES**

(57) Methods, systems and apparatus are described for estimating titer of immunoglobulin single variable domains (ISVDs). The method includes: receiving an ISVD sequence dataset comprising data representative of at least one ISVD sequence; processing the received ISVD sequence dataset with a protein large language model configured for generating an ISVD embeddings dataset; processing the ISVD embeddings dataset with a ML model configured for generating an estimated ISVD titer value for each ISVD sequence of the ISVD sequence dataset based on the ISVD embeddings dataset input; and outputting data representative of the ISVD titer values corresponding to the ISVD sequence dataset for downstream processing.

FIG. 1A

## Description

### Field

This specification relates to methods, systems, and apparatus for estimating titer of "immunoglobulin single variable domain" molecules (ISVDs) from ISVD sequence datasets for use in downstream analysis including *in silica, in vitro* and/or *in vivo* analysis and drug discovery and optimization.

### Background

The term "immunoglobulin single variable domain" (ISVD), interchangeably used with "single variable domain", defines immunoglobulin molecules wherein the antigen binding site is present on, and formed by, a single immunoglobulin domain. This sets immunoglobulin single variable domains apart from "conventional" immunoglobulins (e.g., monoclonal antibodies) or their fragments (such as Fab, Fab', F(ab')$_2$, scFv, di-scFv), wherein two immunoglobulin domains, in particular two variable domains, interact to form an antigen binding site. Typically, in conventional immunoglobulins, a heavy chain variable domain ($V_H$) and a light chain variable domain ($V_L$) interact to form an antigen binding site. In this case, the complementarity determining regions (CDRs) of both $V_H$ and $V_L$ will contribute to the antigen binding site, i.e. a total of 6 CDRs will be involved in antigen binding site formation.

In contrast, ISVDs are capable of specifically binding to an epitope of the antigen without pairing with an additional ISVD. The binding site of an ISVD is formed by a single $V_H$, a single $V_{HH}$ or single $V_L$ domain. Hence, the antigen binding site of an ISVD is formed by no more than three CDRs.

As such, the single variable domain may be a light chain variable domain sequence (e.g., a $V_L$-sequence) or a suitable fragment thereof; or a heavy chain variable domain sequence (e.g., a $V_H$-sequence or $V_{HH}$ sequence) or a suitable fragment thereof; as long as it is capable of forming a single antigen binding unit (i.e., a functional antigen binding unit that essentially consists of the single variable domain, such that the single antigen binding domain does not need to interact with another variable domain to form a functional antigen binding unit).

An ISVD can for example be a heavy chain ISVD, such as a $V_H$, $V_{HH}$, including a camelized $V_H$ or humanized $V_{HH}$. In one embodiment, it is a $V_{HH}$, including a camelized $V_H$ or humanized $V_{HH}$. Heavy chain ISVDs can be derived from a conventional four-chain antibody or from a heavy chain antibody.

For example, the ISVD may be a (single) domain antibody (or an amino acid sequence that is suitable for use as a single domain antibody), a "dAb" or dAb (or an amino acid sequence that is suitable for use as a dAb) or a Nanobody® ISVD (as defined herein and including but not limited to a $V_{HH}$); other single variable domains, or any suitable fragment of any one thereof.

In particular, the ISVD may be a Nanobody® ISVD (such as a $V_{HH}$, including a humanized $V_{HH}$ or camelized $V_H$) or a suitable fragment thereof.

"$V_{HH}$ domains", also known as $V_{HH}$s, $V_{HH}$ antibody fragments and $V_{HH}$ immunoglobulins, have originally been described as the antigen binding immunoglobulin variable domain of "heavy chain antibodies" (i.e., of "antibodies devoid of light chains"; Hamers-Casterman et al. 1993 (Nature 363: 446-448). The term "$V_{HH}$ domain" has been chosen to distinguish these variable domains from the heavy chain variable domains that are present in conventional 4-chain antibodies (which are referred to herein as "$V_H$ domains") and from the light chain variable domains that are present in conventional 4-chain antibodies (which are referred to herein as "$V_L$ domains"). For a further description of $V_{HH}$'s, reference is made to the review article by Muyldermans 2001 (Reviews in Molecular Biotechnology 74: 277-302).

For the term "dAb's" and "domain antibody", reference is for example made to Ward et al. 1989 (Nature 341: 544), to Holt et al. 2003 (Trends Biotechnol. 21: 484); as well as to for example WO 2004/068820, WO 2006/030220, WO 2006/003388 and other published patent applications of Domantis Ltd. It should also be noted that, although less preferred in the context of the present invention because they are not of mammalian origin, single variable domains can be derived from certain species of shark (for example, the so-called "IgNAR domains", see for example WO 2005/18629).

ISVD sequences of different origin, comprising mouse, rat, rabbit, donkey, human and camelid ISVD sequences can be used herein. Also, fully human, humanized, or chimeric sequences can be used in the method described herein. For example, camelid ISVD sequences and humanized camelid ISVD sequences, or camelized domain antibodies, e.g. camelized dAb as described by Ward et al. 1989 (Nature 341: 544), WO 1994/04678, and Davis and Riechmann (1994, Febs Lett., 339:285-290; and 1996, Prot. Eng., 9:531-537) can be used herein. Moreover, the ISVDs are fused forming a multivalent and/or multispecific construct (for multivalent and multispecific polypeptides containing one or more $V_{HH}$ domains and their preparation, reference is also made to Conrath et al. 2001 (J. Biol. Chem., Vol. 276, 10. 7346-7350) as well as to for example WO 1996/34103 and WO 1999/23221).

A "humanized $V_{HH}$" comprises an amino acid sequence that corresponds to the amino acid sequence of a naturally occurring $V_{HH}$ domain, but that has been "humanized", i.e. by replacing one or more amino acid residues in the amino acid sequence of said naturally occurring $V_{HH}$ sequence (and in particular in the framework sequences) by one or more of the amino acid residues that occur at the corresponding position(s) in a $V_H$ domain from a conventional 4-chain antibody from a human being (e.g. indicated above). This can be performed in a manner known per se, which will be clear to the skilled person, for example on the basis of the prior art (e.g. WO 2008/020079). Again, it should be noted that such humanized $V_{HH}$s can be obtained in any suitable manner known per se and thus are not strictly limited to polypeptides that have been obtained using a polypeptide that comprises a naturally occurring VHH domain as a starting material.

A "camelized $V_H$" comprises an amino acid sequence that corresponds to the amino acid sequence of a naturally occurring $V_H$ domain, but that has been "camelized", i.e. by replacing one or more amino acid residues in the amino acid sequence of a naturally occurring $V_H$ domain from a conventional 4-chain antibody by one or more of the amino acid residues that occur at the corresponding position(s) in a $V_{HH}$ domain of a (camelid) heavy chain antibody. This can be performed in a manner known per se, which will be clear to the skilled person, for example on the basis of the description in the prior art (e.g. Davies and Riechman 1994, FEBS 339: 285; 1995, Biotechnol. 13: 475; 1996, Prot. Eng. 9: 531; and Riechman 1999, J. Immunol. Methods 231: 25). Such "camelizing" substitutions are inserted at amino acid positions that form and/or are present at the $V_H$-$V_L$ interface, and/or at the so-called Camelidae hallmark residues, as defined herein (see for example WO 1994/04678 and Davies and Riechmann (1994 and 1996, supra). In one embodiment, the $V_H$ sequence that is used as a starting material or starting point for generating or designing the camelized $V_H$ is a $V_H$ sequence from a mammal, such as the $V_H$ sequence of a human being, such as a $V_H3$ sequence. However, it should be noted that such camelized $V_H$ can be obtained in any suitable manner known per se and thus are not strictly limited to polypeptides that have been obtained using a polypeptide that comprises a naturally occurring $V_H$ domain as a starting material.

The structure of an ISVD sequence can be considered to be comprised of four framework regions ("FRs"), which are referred to in the art and herein as "Framework region 1" ("FR1"); as "Framework region 2" ("FR2"); as "Framework region 3" ("FR3"); and as "Framework region 4" ("FR4"), respectively; which framework regions are interrupted by three complementary determining regions ("CDRs"), which are referred to in the art and herein as "Complementarity Determining Region 1" ("CDR1"); as "Complementarity Determining Region 2" ("CDR2"); and as "Complementarity Determining Region 3" ("CDR3"), respectively.

Generally, Nanobody® ISVDs (in particular VHH sequences, including (partially) humanized VHH sequences and camelized VH sequences) can be characterized by the presence of one or more "Hallmark residues" (as described herein) in one or more of the framework sequences (again as further described herein). Thus, generally, a Nanobody® ISVD can be defined as an immunoglobulin sequence with the (general) structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which one or more of the Hallmark residues are as further defined herein.

In particular, a Nanobody® ISVD can be an immunoglobulin sequence with the (general) structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which the framework sequences are as further defined herein.

More in particular, a Nanobody® ISVD can be an immunoglobulin sequence with the (general) structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which:

one or more of the amino acid residues at positions 11, 37, 44, 45, 47, 83, 84, 103, 104 and 108 according to the Kabat numbering are chosen from the Hallmark residues mentioned in Table A below.

Table A: Hallmark Residues in Nanobody® ISVDs

| Position | Human $V_H3$ | Hallmark Residues |
|---|---|---|
| 11 | L, V; predominantly L | L, S, V, M, W, F, T, Q, E, A, R, G, K, Y, N, P, I; preferably L |
| 37 | V, I, F; usually V | F[1], Y, V, L, A, H, S, I, W, C, N, G, D, T, P, preferably F[1] or Y |
| 44[8] | G | E[3], Q[3], G[2], D, A, K, R, L, P, S, V, H, T, N, W, M, I; preferably G[2], E[3] or Q[3]; most preferably G[2] or Q[3] |
| 45[8] | L | L[2], R[3], P, H, F, G, Q, S, E, T, Y, C, I, D, V; preferably L[2] or R[3] |
| 47[8] | W, Y | F[1], L[1] or W[2] G, I, S, A, V, M, R, Y, E, P, T, C, H, K, Q, N, D; preferably W[2], L[1] or F[1] |
| 83 | R or K; usually R | R, K[5], T, E[5], Q, N, S, I, V, G, M, L, A, D, Y, H; preferably K or R; most preferably K |

(continued)

| Position | Human $V_H3$ | Hallmark Residues |
|---|---|---|
| 84 | A, T, D; predominantly A | P[5], S, H, L, A, V, I, T, F, D, R, Y, N, Q, G, E; preferably P |
| 103 | W | W[4], R[6], G, S, K, A, M, Y, L, F, T, N, V, Q, P[6], E, C; preferably W |
| 104 | G | G, A, S, T, D, P, N, E, C, L; preferably G |
| 108 | L, M or T; predominantly L | Q, L[7], R, P, E, K, S, T, M, A, H; preferably Q or L[7] |

Notes:

(1) In particular, but not exclusively, in combination with KERE or KQRE at positions 43-46.

(2) Usually as GLEW at positions 44-47.

(3) Usually as KERE or KQRE at positions 43-46, e.g. as KEREL, KEREF, KQREL, KQREF, KEREG, KQREW or KQREG at positions 43-47. Alternatively, also sequences such as TERE (for example TEREL), TQRE (for example TQREL), KECE (for example KECEL or KECER), KQCE (for example KQCEL), RERE (for example REREG), RQRE (for example RQREL, RQREF or RQREW), QERE (for example QEREG), QQRE, (for example QQREW, QQREL or QQREF), KGRE (for example KGREG), KDRE (for example KDREV) are possible. Some other possible, but less preferred sequences include for example DECKL and NVCEL.

(4) With both GLEW at positions 44-47 and KERE or KQRE at positions 43-46.

(5) Often as KP or EP at positions 83-84 of naturally occurring $V_{HH}$ domains.

(6) in particular, but not exclusively, in combination with GLEW at positions 44-47.

(7) With the proviso that when positions 44-47 are GLEW, position 108 is always Q in (non-humanized) $V_{HH}$ sequences that also contain a W at 103.

(8) The GLEW group also contains GLEW-like sequences at positions 44-47, such as for example GVEW, EPEW, GLER, DQEW, DLEW, GIEW, ELEW, GPEW, EWLP, GPER, GLER and ELEW.

In one embodiment, the ISVD has certain amino acid substitutions in the framework regions effective in preventing or reducing binding of so-called "pre-existing antibodies" to the polypeptides. ISVDs in which (i) the amino acid residue at position 112 is one of K or Q; and/or (ii) the amino acid residue at position 89 is T; and/or (iii) the amino acid residue at position 89 is L and the amino acid residue at position 110 is one of K or Q; and (iv) in each of cases (i) to (iii), the amino acid at position 11 is preferably V have been described in WO2015/173325.

ISVDs allow a broad range of applications in biotechnical as well as therapeutic use due to their small size, simple production, and high affinity. The titer of ISVDs can be determined using various laboratory methods, such as, without limitation, for example with spectrophotometry (A280), Bradford assay, Bicinchoninic Acid (BCA) assay, enzyme-linked immunosorbent assay (ELISA), or Sodium Dodecyl Sulfate-Polyacrylamide Gel Electrophoresis (SDS-PAGE)/Western blot among others. The choice of method depends on the available equipment, the required sensitivity, and the specific characteristics of the ISVDs being measured. The many techniques of measuring ISVD titer usually require wet lab analysis techniques such as, for example, ELISA or specifically requires developing an ELISA-type test depending on the particular ISVD and the like. This can be time consuming, expensive and complicated especially with a large number of ISVDs. There is a desire for performing ISVD titer estimations *in-silico* from ISVD sequence datasets.

**Summary**

According to a first aspect, there is provided a computer-implemented method for estimating a titer of immunoglobulin single variable domains (ISVDs) the method comprising: receiving an ISVD sequence dataset comprising data representative of at least one ISVD sequence; processing the received ISVD sequence dataset with a protein large language embeddings model configured for generating an ISVD embeddings dataset; processing the ISVD embeddings dataset with a machine learning, ML, model configured for generating an estimated ISVD titer value for each ISVD sequence of the ISVD sequence dataset based on the ISVD embeddings dataset input; and outputting data representative of the ISVD titer values corresponding to the ISVD sequence dataset for downstream processing.

As an option, the computer-implemented method, wherein each ISVD sequence comprises an amino acid sequence of an ISVD molecule. As another option, the computer-implemented method, wherein the ISVD sequence dataset comprises a plurality of monovalent ISVDs with different amino acid sequence lengths. In a further option, the computer-implemented method, wherein the ISVD sequence dataset comprises a plurality of multivalent ISVDs with different amino acid sequence lengths.

Optionally, the computer-implemented method, wherein the ISVD sequence dataset comprises a plurality of different monovalent and multivalent ISVDs with different amino acid sequence lengths.

The computer-implemented method, wherein the multivalent ISVDs comprise at least one or more from the group of: bivalent ISVDs; trivalent ISVDs; tetravalent ISVDs; pentavalent ISVDs; hexavalent ISVDs; V-valency ISVDs, where V>1.

The computer-implemented method, wherein the ML model is an ML classifier configured for classifying each ISVD sequence of the ISVD sequence dataset with an ISVD titer class from a set of ISVD titer class labels based on the ISVD embeddings dataset as input, and said processing the ISVD embeddings dataset further comprising inputting the ISVD embeddings for each ISVD sequence to the ML classifier for classifying said each ISVD sequence with an ISVD titer class from the set of ISVD class labels, wherein the ISVD titer class is the generated estimated ISVD titer value.

The computer-implemented method, wherein the set of ISVD titer class labels comprises at least a low titer class label and a high titer class label, wherein the low titer class label corresponds to ISVD sequences classified as having a titer value less than a first threshold titer value, and the high titer class label corresponds to ISVD sequences classified as having a titer value greater than or equal to a second threshold titer value, wherein the first threshold titer value is less than or equal to the second threshold titer value.

The computer-implemented method, wherein the ML classifier is a binary classifier, and the first and second threshold titer values are the same.

The computer-implemented method, wherein the ML classifier is a multi-class classifier, and the set of ISVD titer class labels comprises at least a low titer class label, a medium titer class label and a high titer class label, wherein the first and second threshold titer values are the different, and the medium titer class label corresponds to ISVD sequences classified as having a titer value between the first and second threshold titer values.

The computer-implemented method, wherein the ML classifier is trained using at least one ML classifier algorithm from the group of: a Boosting ML classifier algorithm comprising one or more of: a AdaBoost classifier algorithm; a LPBoost classifier algorithm; a TotalBoost classifier algorithm; a BrownBoost classifier algorithm; a XGBoost classifier algorithm; a MadaBoost classifier algorithm; a LogiBoost classifier algorithm; and any other suitable Boosting ML classifier algorithm suitable for classifying each ISVD sequence of the ISVD sequence dataset with an ISVD titer class from the set of ISVD titer class labels based on the ISVD embeddings dataset as input; a decision-tree based classifier algorithms comprising one or more of: a Random Forest based classifier algorithm; a Extra Trees based classifier algorithm;

any other suitable decision-tree based classifier algorithm suitable for classifying each ISVD sequence of the ISVD sequence dataset with an ISVD titer class from the set of ISVD titer class labels based on the ISVD embeddings dataset as input; a Naive Bayes, NB, based classifier algorithm comprising one or more of: a Gaussian NB based classifier algorithm; any other suitable NB based classifier algorithm suitable for classifying each ISVD sequence of the ISVD sequence dataset with an ISVD titer class from the set of ISVD titer class labels based on the ISVD embeddings dataset as input; an Artificial Neural Network, ANN, based classifier algorithm including one or more of: feed-forward NN based classifier algorithm;
convolutional based classifier algorithm; a recurrent network based classifier algorithm;
a graph neural network algorithm and any other suitable ANN based classifier algorithm suitable for classifying each ISVD sequence of the ISVD sequence dataset with an ISVD titer class from the set of ISVD titer class labels based on the ISVD embeddings dataset as input; a K-nearest Neighbour based classifier algorithm; a Logistic Regression based classifier algorithm;
a Ridge regression based classifier algorithm; a support vector based classifier algorithm;
a stochastic gradient descent, SGD, based classifier algorithm; any other suitable ML classifier algorithm suitable for classifying each ISVD sequence of the ISVD sequence dataset with an ISVD titer class from the set of ISVD titer class labels based on the ISVD embeddings dataset as input.

As an option, the computer-implemented method, wherein the ML model is a trained regression ML model configured for estimating a ISVD titer value for each ISVD sequence of the ISVD sequence dataset based on the ISVD embeddings dataset as input.

Optionally, the computer-implemented method, wherein the ML model is trained based on a training ISVD dataset comprising a plurality of training data instances, each training data instance comprising data representative of an ISVD sequence and a corresponding titer value associated with the ISVD sequence.

As an option, the computer-implemented method further comprising: generating the training ISVD dataset by: receiving a ISVD sequence dataset with associated titer values for each ISVD sequence in the ISVD sequence dataset; processing the ISVD sequence dataset with the protein large language embeddings model to generate ISVD embeddings for each ISVD sequence; associating the ISVD embeddings for each ISVD sequence with the corresponding titer value for said each ISVD sequence; and outputting the training ISVD dataset, wherein each training ISVD data instance comprises the ISVD embeddings for each ISVD sequence and the corresponding titer value.

As another option, the computer-implemented method, wherein when the ML model is to be trained as an ML classifier model, training said ML classifier model by: annotating the training ISVD dataset based on labelling each ISVD training data instance with a class label from a set of class labels based on the corresponding titer value and the corresponding threshold titer values associated with each class label; and training the ML classifier model based on the annotated training ISVD dataset.

As an option, the computer-implemented method, wherein said training further comprising: iteratively performing leave one out cross validation training of the ML model based on using an ML algorithm to train model parameters defining the ML model for classifying the ISVD titer value based on the training ISVD dataset; determining, after all iterations of leave one out cross validation training have been performed, whether said leave one out cross validation training yielded valid ML classification results; and in response to determining whether the leave one out cross validation yielded valid results, training a final ML model using said ML algorithm and the entire training ISVD dataset to train model parameters defining the final ML model for classifying the ISVD titer value.

Optionally, the computer-implemented method, wherein the downstream processing comprises generating a shortlist of ISVD sequences based on those ISVD sequences in the received ISVD sequence dataset with an estimated ISVD titer value that meet a specified titer criteria.

As an option, the computer-implemented method further comprising expressing the ISVD molecules on the shortlist in vitro based on the interaction of one or more compounds or molecules resulting in expression of the ISVD molecules, and selecting the one or more compounds and/or ISVD molecules in the shortlist for further analysis associated with *in vivo* trials, *in vitro* wet lab analysis, and/or high-throughput screening laboratory analysis.

As another option, the computer-implemented method of any preceding claim, further comprising: generating a plurality of ISVD sequences *in silico* as the ISVD sequence dataset, wherein said protein large language embedding model and ML model processes the ISVD sequence dataset to predict or estimate an ISVD titer value for each of ISVD sequences in the ISVD sequence dataset; and generating a shortlist of ISVD sequences based on selecting ISVD sequences from the ISVD sequence dataset with predicted or estimated ISVD titer values meeting predetermined titer criteria; outputting the shortlist of ISVD sequences for ISVD sequence production of said ISVD sequences and subsequent *in vitro* analysis, assays, and/or high-throughput screening laboratory analysis.

As a further option, the computer-implemented method of any preceding claim, wherein the ISVD embeddings for each ISVD sequence are in the form of a high dimensional vector of fixed length $N$, where $N >> 0$ is the total number of ISVD embeddings used for representing each ISVD sequence.

Optionally, the computer-implemented method, wherein the protein large language embedding model comprises a protein large language model configured for outputting, for each ISVD sequence that is input, a sequence of M embeddings vectors, each embeddings vector of fixed size N, and said protein large language embedding model is further configured for outputting a "mean" representations embeddings vector of fixed size N as the ISVD embeddings for said each ISVD sequence based on computing an average of the corresponding sequence of $M$ embeddings vectors.

As an option, the computer-implemented method, wherein the protein large language model is a pre-trained transformer protein language model configured to receive a variable sized input vector representing each ISVD sequence and further configured to generate an output representative of the ISVD embeddings.

Optionally, the computer-implemented method, wherein the protein large language model is a pre-trained neural network based protein language model configured to receive a variable sized input vector representing an ISVD sequence and configured to generate a fixed length output vector of length $N$, where $N>>0$, representing the ISVD embeddings.

As another option, the computer-implemented method, wherein the protein large language model is configured to output a selected hidden layer and generate data representative of the ISVD embeddings.

As an option, the computer-implemented method, wherein the protein large language model further comprises one or more protein large language models from the group of: an Evolutionary Scale Model, ESM, 1 based protein large language model; an Evolutionary Scale Model, ESM, 2 based protein large language model; any other type of protein large language model configured to receive data representative of an ISVD sequence representing $M$ amino acids, $M>>0$, as input and generate a sequence of $M$ embeddings vectors as output; and any other type of protein large language model configured to receive an ISVD sequence as input and generate the ISVD embeddings as output.

As another option, the computer-implemented method, wherein the ESM based protein large language model further comprises one or more from the group of: esm1_t34_670M_UR50S; esm1b t33 650M_UR50S; esm1v_t33_650M_UR90S_1; esm2_t36_3B_UR50D; any other ESM based protein large language model configured to receive data representative of an ISVD sequence representing $M$ amino acids, $M>>0$, as input and generate a sequence of $M$ embeddings vectors as output; and any other ESM based protein large language model configured to receive an ISVD sequence as input and generate the ISVD embeddings as output.

Optionally, the computer-implemented method, wherein the ISVD sequence dataset is based on the amino acid sequence described in any type of annotation scheme or format selected from one or more of the Fast-All (FASTA) format, International ImMunoGeneTics Information System (IMGT) annotation scheme, the Kabat annotation scheme, the Chothia annotation scheme, the Martin annotation scheme, the Wolfguy annotation scheme, or the AHo annotation scheme, and/or any other type of annotation scheme or format suitable for describing ISVDs.

According to a second aspect, there is provided an apparatus comprising a processor, a memory unit, and a communication interface, wherein the processor is connected to the memory unit and the communication interface, wherein the processor and memory are configured to implement the computer-implemented method of the first aspect and any one or more features thereof.

According to a third aspect, there is provided a computer program product comprising data or instruction code, which when executed on a processor, causes the processor to implement the computer-implemented method of the first aspect and any one or more features thereof.

According to a fourth aspect, there is provided a computer-readable medium comprising data or instruction code, which when executed on a processor, causes the processor to implement the computer-implemented method of the first aspect and any one or more features thereof.

According to a fifth aspect, there is provided a non-transitory tangible computer-readable medium comprising data or instruction code there is provided a computer-implemented method for estimating a titer of immunoglobulin single variable domains (ISVDs) the method comprising: receiving an ISVD sequence dataset comprising data representative of at least one ISVD sequence; processing the received ISVD sequence dataset with a protein large language embeddings model configured for generating an ISVD embeddings dataset; processing the ISVD embeddings dataset with a machine learning, ML, model configured for generating an estimated ISVD titer value for each ISVD sequence of the ISVD sequence dataset based on the ISVD embeddings dataset input; and outputting data representative of the ISVD titer values corresponding to the ISVD sequence dataset for downstream processing.

According to a sixth aspect, there is provided a computing system comprising one or more processors coupled to a memory, wherein the one or more processors and memory are configured to implement the computer-implemented method of the first aspect and any one or more features thereof.

In various implementations, there is provided computer program instructions or code, optionally stored on a non-transitory computer readable medium which, when executed by one or more processors of a data processing apparatus, causes the data processing apparatus to carry out the program instructions to cause the one or more processors to perform operations comprising one or more aspects of the above- and/or below-described implementations (including one or more aspects of the appended claims).

In various implementations, there is provided a computer program product comprising computer program instructions or code which, when executed by one or more processors of a data processing apparatus, causes the data processing apparatus to carry out the program instructions to cause the one or more processors to perform operations comprising one or more aspects of the above- and/or below-described implementations (including one or more aspects of the appended claims).

In various implementations, apparatus are disclosed that comprise a non-transitory computer readable storage medium having program instructions embodied therewith, and one or more processors configured to execute the program instructions to cause the apparatus to perform operations comprising one or more aspects of the above- and/or below-described implementations (including one or more aspects of the appended claims). The apparatus may comprise one or more processors or special-purpose computing hardware.

**Brief Description of the Drawings**

So that the invention may be more easily understood, embodiments thereof will now be described by way of example only, with reference to the accompanying drawings in which:

Figure 1a illustrates an example ISVD titer prediction system according to some embodiments of the invention;

Figure 1b illustrates an example ISVD titer prediction process according to some embodiments of the invention;

Figure 1c illustrates an example ISVD titer training process according to some embodiments of the invention;

Figure 2a illustrates another example ISVD titer classifier training process according to some embodiments of the invention;

Figure 2b illustrates another example ISVD titer regressor training process according to some embodiments of the invention;

Figure 3a illustrates an example ISVD dataset for use in the training ISVD model process of Figures 1a to 2a according to some embodiments of the invention;

Figure 3b illustrates the example ISVD dataset of Figure 3a for different ranges of titer thresholds according to some embodiments of the invention;

Figure 4 illustrates example titer classifier models trained for binary titer classification using the ISVD dataset of Figures 3a and 3b according to some embodiments of the invention;

Figure 5 illustrates example boxenplot and swarmplot performance results for example trained titer classification models of Figure 4 when predicting titer values for different valency ISVDs;

Figure 6 is a schematic illustration of a system/apparatus for performing the methods/processes described herein;

Common reference numerals are used throughout the figures to indicate similar features.

## Detailed Description

Various example implementations described herein relate to method(s), apparatus, and system(s) for automatically, efficiently, and reliably generating *in silica* titer estimations of ISVD sequences for use in downstream biological and/or pharmacological analyses, processes, and/or applications. In some examples, the ISVD sequences may form an ISVD sequence dataset that has a plurality of ISVD sequences associated with different monovalent ISVDs having different amino acid sequence lengths. In other examples, the ISVD sequences may form an ISVD sequence dataset that has a plurality of ISVD sequences associated with different multivalent ISVDs having different amino acid sequence lengths. In further examples, the ISVD sequences may form an ISVD sequence dataset that has a plurality of ISVD sequences associated with different monovalent and multivalent ISVDs and having different amino acid sequence lengths. The multivalent ISVDs may include, without limitation, for example, one or more from the group of: bivalent ISVDs; trivalent ISVDs; tetravalent ISVDs; pentavalent ISVDs; and hexavalent ISVDs, and/or any V-valent ISVDs, V>1 and the like.

An ISVD titer model is trained and configured for predicting ISVD titer values when high-dimensional embeddings of an ISVD sequence is input. The ISVD titer model may be trained to predict titer values for a single valency type, without limitation, for example, only monovalent ISVDs, only bivalent ISVDs; only trivalent ISVDs; only tetravalent ISVDs; only pentavalent ISVDs; and only hexavalent ISVDs, and the like. Alternatively, the ISVD titer model may be trained to predict titer values for various different valency types such as, without limitation, two or more of monovalent ISVDs, bivalent ISVDs; trivalent ISVDs; tetravalent ISVDs; pentavalent ISVDs; and hexavalent ISVDs, and/or any V-valent ISVDs, V>0 and the like.

Although the ISVD titer model and inference/prediction results thereof are described herein in relation to monovalent ISVDs up to hexavalent ISVDs, this is by way of example only and the ISVD titer model is not so limited. It is to be appreciated by the skilled person that the techniques, methods, apparatus, and systems described herein for generating, training, and/or operating the ISVD titer model are applicable, subject to using suitable ISVD labelled training datasets, to higher multivalency ISVDs such as, without limitation, for example heptavalent ISVDs, octavalent ISVDs, nonavalent ISVDs, decavalent ISVD, and V-valency ISVDs for V>1 and beyond, and/or any suitable higher multivalency ISVDs as the application demands.

In general, there is a scarcity of ISVD labelled datasets for each of the different valencies of ISVD sequences, which poses challenges for validly training an ISVD titer model. However, this scarcity of data is alleviated as described herein by the judicious pairing of protein large language model embeddings with machine learning algorithms for training one or more ISVD titer models that have the capability to accurately predict the titer value/class of unknown ISVD sequences for different valencies and/or generalised to all valencies. It has been found that an ISVD dataset labelled with known titer values containing different valency ISVD sequences can be used to validly train an ISVD titer model that is capable of predicting ISVD titer for unknown ISVD sequences with unknown titer and having different valencies. This is a bonus technical effect because, in general, training an ML model on a dataset including different molecule types from the molecule of interest often does not improve predictive accuracy when predicting properties for each of the particular types of molecules. It has further been found that, even though there is a scarcity of ISVD data labelled with known titer values associated with a particular valency, the pairing as described herein also results in ISVD titer models, which once trained on the scarce ISVD data associated with the particular valency, are capable of reliably predicting ISVD titer for unknown ISVD sequences of the particular valency. This is another bonus technical effect because, in general, the accuracy of an ML model and its predictive capability typically depends on the size of the training dataset.

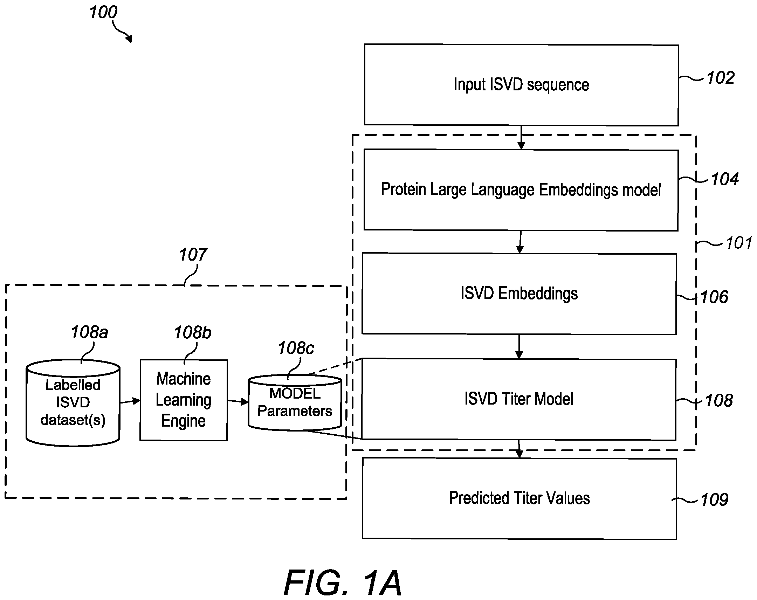

Figure 1a shows an example of a ISVD titer estimation system 100. The system 100 is an example of a system implemented as computer programs on one or more computers in one or more locations, in which the systems, components, and techniques described below can be implemented.

The ISVD titer estimation system 100 uses a machine-learning model 101 to predict ISVD titer values 109 of ISVDs based on an input ISVD sequence dataset 102 specifying one or more ISVD sequences. An ISVD sequence of the ISVD sequence dataset 102 includes data representative of an amino acid sequence of the ISVD corresponding to the ISVD sequence. The ISVD sequence dataset 102 may include a plurality of ISVD sequences, each ISVD sequence being unique. Alternatively, the ISVD sequence dataset 102 includes one ISVD sequence.

In general, the machine-learning model 101 includes a protein large language embeddings model 104 configured for generating a set of embeddings associated with each ISVD sequence in the ISVD sequence dataset, and an ISVD titer machine-learning model 108 (also referred to as a ISVD titer model) having a set of model parameters 108c configured for predicting a ISVD titer value from each set of embeddings 106 for a corresponding ISVD sequence 102.

The protein large language embeddings model 104 is configured to receive a variable length input vector comprising data representative of an amino acid sequence of an ISVD and generate a set of embeddings. The set of embeddings may be represented in the form of a fixed length embedding vector that is much larger than the variable length input vector.

The variable length input vector may include the amino acid sequence data from any appropriate annotation scheme or format used to generate the annotations of the amino acid sequence of each ISVD. Examples of annotation schemes or formats include, without limitation, for example the Simple format, Fast-All (FASTA) format, International ImMunoGeneTics Information System (IMGT), Kabat, Chothia, Martin, Wolfguy, and AHo annotation schemes or formats. The input ISVD sequence 102 is a vector representing the amino acid sequence represented within the annotation scheme or format used to describe the ISVD. Only the amino acid sequence of an ISVD represented in a particular annotation scheme or format is processed by the protein large language model.

The protein large language embeddings model 104 is configured to process the input ISVD sequence vector 102 representation of an ISVD sequence and generate a corresponding sequence of tokens (per token representation), in which each token in the sequence of tokens is a high dimensional vector of a fixed length, $N, N \gg 0$ (e.g., $N = 512, 1024, 2048,$ or $4096$ etc.) representing embeddings of a corresponding amino acid of the ISVD sequence. The sequence of tokens is referred to as a per token representation from which a mean representation is computed to generate a vector of mean embeddings of fixed length $N$. The mean representation is computed by averaging the tokens of the sequence of tokens together to form a single high dimensional vector of mean embeddings (also referred to as a mean embeddings vector) that has a fixed length $N$. The mean embeddings may also be referred to herein as descriptors or protein-descriptors. The protein large language embeddings model 104 outputs, for each ISVD sequence that is input, a single high dimensional mean embeddings vector as ISVD embeddings 106.

The protein large language embeddings model 104 is configured to receive any variable length input sequence vector 102 representing an ISVD sequence and transforms the input sequence vector 102 into a corresponding high dimensional protein-descriptor vector of fixed length $N$ representing the mean embeddings of the ISVD sequence. For example, an ISVD dataset comprising a plurality of variable length ISVD sequences can be transformed by the protein large language embeddings model 104 into a corresponding ISVD embeddings dataset 106 comprising a corresponding plurality of mean embeddings vectors of fixed length $N$.

In an example embodiment, the protein large language embeddings model 104 is configured to generate a sequence of tokens (or per token representations) for an ISVD sequence. Each token refers to the distinct embeddings that the protein large language embeddings model 104 generates for each individual amino acid in an ISVD sequence. That is, the embeddings generated by the protein large language embeddings model 104 for each individual amino acid are called a token (per token representation), which takes the form of a high dimensional vector of a fixed length, $N, N \gg 0$. Thus, for each amino acid of an ISVD sequence, the protein large language embeddings model 104 generates a distinct high dimensional vector of length $N$ (a token) of embeddings, which are real numbers.

Each token represents a vector of embeddings that capture contextual information specific to the amino acid it represents within the ISVD sequence. These token embeddings can be used for understanding or predicting properties of individual amino acids or when detailed positional information within an ISVD sequence is required. However, for classification or regression tasks the mean representation can be used. The mean representation for an ISVD sequence is a single high dimensional vector of fixed length $N$ of mean embeddings (i.e., a mean embeddings vector) that summarizes the entire ISVD sequence. The mean representation is computed or derived by averaging the "per token" embeddings across the token sequence generated by the protein large language embeddings model 104. That is the embeddings vectors representing the tokens within the token sequence are averaged together to form a single high dimensional mean embeddings vector of fixed length $N$. The protein large language embeddings model 104 outputs, for each ISVD sequence that is input, a single high dimensional mean embeddings vector as ISVD embeddings 106. The mean representation provides a holistic view of the ISVD sequence and is useful for tasks where an ISVD sequence needs to be considered as a whole. It has been found that the ISVD embeddings 106 for an ISVD sequence is a numerical representation of input the data that captures the essential contextual information or features associated with ISVD sequences enabling the prediction of titer of ISVD sequences.

As a hypothetical example, consider an ISVD sequence of $M$ amino acids: "ACDE......G", $M > 0$. Suppose the protein large language model of the protein large language embeddings model generates a sequence of tokens for this ISVD sequence, where each token represents each amino acid and is an $N$-dimensional embeddings vector, where $N \gg 0$

(e.g., $N$ = {256, 512, 1024, 2048, 4096, etc.}). In this example, the ISVD sequence of amino acids is processed where the protein large language model generates the following sequence of tokens of:

- A (Alanine position 1): v1 = [0.2,0.5,... ..., 0.3]

- C (Cysteine position 2): v2 = [0.1,0.4,... ..., 0.6]

- D (Aspartic acid position 3): v3 = [0.3,0.8,... ...,0.7]

- E (Glutamic acid position 4): v4 = [0.4,0.7,... ...,0.2]

- G (Glycine position $M$): v$M$ = [0.6,0.3,... ...,0.9]

The sequence of embeddings vectors v1, v2, v3, v4,... ...v$M$ are the "per token" representations, each vector corresponding to a unique set of embeddings for a corresponding individual amino acid in the ISVD sequence. Each position and amino acid are represented by a unique embeddings vector, which represents that particular amino acid, position, and so-called "context" or contextual information. The protein large language model generates these embeddings vectors in a way that captures the contextual information of the amino acids in the ISVD sequence, so even if the same amino acid appears multiple times in an ISVD sequence, its "per token" representation can vary based on its position and surrounding context.

Even though the per tokens representation may be a sequence of $M$ vectors, each of vector size $N$, the per tokens representation may also be represented as an $M \times N$ "per tokens" representation matrix, denoted **pT,** where $M$ is the Protein length (variable based upon the input ISVD sequence) and $N$ is the size of the embeddings vector (a constant when using the same protein large language model, which depends on how the model was trained and the output selected). The $m$-th row of the "per tokens" representation matrix, **pT,** is the $m$-th embeddings vector v$m$ representing the $m$-th amino acid of the corresponding ISVD sequence, where $1 \leq m \leq M$. For example, in the case of ESM2 protein large language models (pLMs), each "per token" representation is represented by an embeddings vector of size $N$ (e.g., $N$ = 1024 or 2048 elements). As an example, with $N$ = 1024, a monovalent ISVD sequence of size $M$ = 120 amino acids, will have a "per tokens" representation matrix size of 120 $\times$ 1024, while a trivalent ISVD sequence of size $M$ = 400 amino acids, will have a "per token" representation matrix size of 400 $\times$ 1024, and so on. In essence, a protein large language model outputs, for each ISVD sequence, a "per tokens" representation matrix, **pT,** in which the number of rows $M$ is the size of the corresponding ISVD sequence and the number of columns $N$ is the size of the embeddings vector generated and output by the protein large language model for each amino acid in the corresponding ISVD sequence. The "per tokens" representation matrix, **pT**, can be used for downstream classification and regression tasks for a protein of interest, while its application is limited to certain ML architectures, including Neural Networks or Graph Neural Networks.

For each ISVD sequence that is processed, the protein large language embeddings model computes, from the "per token" representation matrix, **pT**, output by the protein large language model for the corresponding ISVD sequence, a "mean" representation embeddings vector of size $N$ by taking the average of all the sequence of embedding vectors v1, v2, v3, v4,......v$M$. That is, the $M$ rows of the "per tokens" representation matrix, **pT**, are averaged together to yield the "mean" representation embeddings vector, **ev**. For example, the n-th element of the "mean" representation embeddings vector ev, for $1 \leq n \leq N$, is computed by $\frac{1}{M} \times \sum_{i=1}^{M} \mathbf{pT}[i,n]$, where pT[l,n] denotes the $i$-th row and $n$-th column of $MxN$ matrix **pT.** For example, in the above example ISVD sequence of ACDE...G, and assuming for simplicity that $M$=5 and $N$=3, then the sequence of embeddings vectors is:

- A (Alanine position 1): v1 = [0.2, 0.5, 0.3]
- C (Cysteine position 2): v2 = [0.1, 0.4, 0.6]
- D (Aspartic acid position 3): v3 = [0.3, 0.8, 0.7]
- E (Glutamic acid position 4): v4 = [0.4, 0.7, 0.2]
- G (Glycine position 5): v5 = [0.6, 0.3, 0.9]

This resulting a "per token" representation matrix, **pT**, of:

$$\mathbf{pT} = \begin{bmatrix} \mathbf{v}1 \\ \mathbf{v}2 \\ \mathbf{v}3 \\ \mathbf{v}4 \\ \mathbf{v}5 \end{bmatrix} = \begin{bmatrix} 0.2 & 0.5 & 0.3 \\ 0.1 & 0.4 & 0.6 \\ 0.3 & 0.8 & 0.7 \\ 0.4 & 0.7 & 0.2 \\ 0.6 & 0.3 & 0.9 \end{bmatrix},$$

in which the "mean embedding" of the first element of the mean representation embeddings vector, **ev**, is the average (or the mean) of the first element of all the "per token" vectors v1, v2, v3, v4 and v5, or the average of the first column of the "per tokens" representation matrix, **pT,** which is: (0.2+0.1+0.3+0.4+0.6)/5 = 0.32. The "mean embedding" of the second element of the mean representation embeddings vector, **ev**, is (0.2+0.1+0.3+0.4+0.6)/5 = 0.32, and the "mean embedding" of the last element of the mean representation embeddings vector, **ev**, is (0.2+0.1+0.3+0.4+0.6)/5 = 0.32. Hence, the mean representation embeddings vector, **ev**, for this simple example is, **ev** = [0.32. 0.54, 0.54].

For each ISVD sequence, the mean representations embeddings vector, **ev**, summarizes the entire ISVD sequence and can be used for tasks where a comprehensive representation of the protein is needed, such as in classification or clustering. Although the above example assumed $M=5$ and $N=3$ resulting in an **ev** vectors of size 3, this is for simplicity and the invention is not so limited, it is to be appreciated by the skilled person that typically $M>>0$ and $N>>0$ resulting in a high dimensional mean representation embeddings vector, **ev**, for each ISVD sequence. For example, for typical ESM2 protein large language models, the size of $N$ may be of a size of 1024, 2048, 4096, or higher depending on the number of parameters of the model.

For example, if the ESM2 protein large language model processes an ISVD sequence and outputs a sequence of embeddings vectors of size $N$ (e.g., $N=1024$), then the mean representation embeddings vector, **ev**, for the ISVD sequence is of size $N$ and is said to have $N$ mean embeddings. These $N$ mean embeddings of an ISVD sequence may be referred to as descriptors, or protein-descriptors, which can be used for ML classification tasks such as, for example, titer prediction/classification.

The "mean" representation allows all ISVD sequences (e.g., Nanobody® ISVDs) to be described with the same number of descriptors or protein-descriptors. For example, a monovalent ISVD sequence of length $M=120$, where in this simple example, $N=1024$, will have a "per token" representation matrix of size $120 \times 1024$ but will have a "mean" representation embeddings vector, **ev**, of size $1 \times 1024$. A multivalent ISVD sequence of length $M=400$, will have a "per token" representation matrix of size $400 \times 1024$, but a "mean" representation embeddings vector, **ev**, of size $1 \times 1024$. It has been found that the advantage of using the "mean" representation is that monovalent and multivalent ISVD sequences and corresponding titer data may be used together for training ML algorithms for predicting titer values or titer classes for unknown ISVD sequences regardless of valency. This enables combining multiple data sources together, i.e., monovalent and multivalent ISVD sequences and titer data, to enhance training of the corresponding ML algorithms for generating ISVD titer models configured for predicting titer for all unknown monovalent or multivalent ISVD sequences.

Thus, the protein large language embeddings model 104 outputs, for each ISVD sequence that is input, a single high dimensional mean representation embeddings vector, **ev**, of size $N$, where $N>>0$, which is also referred to as the ISVD embeddings 106.

In some implementations, the protein large language embeddings model 104 is based on a neural network structure. The neural network structure may include an input layer, one or more hidden layers, and an output layer. The input layer receives the ISVD sequence 102 and the hidden layers process this data with the output layer outputting the sequence of tokens and configured to compute the mean representation and corresponding ISVD embeddings 106. Alternatively, depending on what the protein large language model is configured to output, ISVD embeddings may be generated from an output of a selected hidden layer of the neural network. The neural network can adopt any appropriate architecture. For example, the neural network can include at least a portion (e.g., the embedding portion) of a state-of-the-art large language model.

For example, in some implementations, the embedding neural network of the protein large language embeddings model 104 may include, without limitation, for example an encoder network of a variational autoencoder (VAE). Implementation examples of a VAE are described in "Auto-encoding variational Bayes,"Kingma et al., arXiv: 1312.6114, 2013.

In some implementations, the neural network of the protein large language embeddings model 104 can include the embedding layers of an autoregressive transformer, e.g., a generative pre-trained transformer (GPT). Implementation examples of the GPT are described in "Language Models are Few-Shot Learners," Brown et al., Advances in Neural Information Processing Systems 33: 1877-1901, 2020.

In some implementations, the neural network of the protein large language embedding model 104 can include a bidirectional transformer, e.g., a bidirectional encoder representations from transformers (BERT) model. Implementation examples of the BERT are described in "BERT: Pre-training of Deep Bidirectional Transformers for Language Understanding," Devlin et al., arXiv:1810.04805, 2018.

In some embodiments, the protein large language embeddings model 104 is a pre-trained neural network based protein language model configured to receive a variable sized input vector representing an ISVD sequence 102 and generate a fixed length output mean embeddings vector of length $N$, where $N>>0$, representing ISVD embeddings 106 associated with the input ISVD sequence. For example, the protein large language embeddings model 104 may include a pre-trained transformer protein language model configured to receive a variable sized input vector representing an ISVD sequence and generate a fixed length output mean embeddings vector of length $N$, where $N>>0$, representing the ISVD embeddings 106 for each input ISVD sequence 102.

In some embodiments, if the protein large language embeddings model 104 is not configured to output an output mean embeddings vector representing ISVD embeddings, the protein large language embeddings model 104 may be further modified or configured to output a selected hidden layer, in which the output of the selected hidden layer is transformed to represent a mean embeddings vector of length $N$, $N \gg 0$, as the ISVD embeddings 106 corresponding to the input ISVD sequence that is processed by the protein large language embeddings model 104.

In some embodiments, the protein large language embeddings model 104 may be a pre-trained protein large language embeddings model that is based on one or more protein large language models from the group of: an Evolutionary Scale Model (ESM)-1 based protein large language model; an ESM-2 based protein large language model; any other type of protein large language model configured to receive data representative of an ISVD sequence as input and generate corresponding sequence of tokens, where a mean representation of the sequence of tokens is computed as described herein to generate the ISVD embeddings 106 for the input ISVD sequence; and/or any other type of protein large language model configured to receive an ISVD sequence as input and generate the ISVD embeddings as output. The ISVD embeddings for an ISVD sequence may be output from a selected hidden layer of the ESM based models.

The ESM based protein large language models that may be used in the protein large language embeddings model 104 may include one or more ESM models from the group of: esm1_t34_670M_UR50S; esm1b_t33_650M_UR50S; esm1v_t33_650M_UR90S_1; esm2_t36_3B_UR50D; and any other ESM based protein large language model configured to receive an ISVD sequence as input and generate either a sequence of tokens that may be aggregated into a mean representation for output as ISVD embeddings; any other ESM based protein large language model configured to receive an ISVD sequence as input and generate a mean representation for output as ISVD embeddings.

Although ESM models are described herein, this is by way of example only and the protein large language embeddings model 104 is not so limited, it is to be appreciated by the skilled person that any suitable type of protein large language embeddings model 104 may use any type of pretrained protein large language model for use in the protein large language embeddings model 104 for generating ISVD embeddings for each ISVD sequence that may be input and/or processed by said protein large language embeddings model 104.

The system 100 further includes an ISVD titer machine-learning model 108 also referred to as an ISVD titer model 108 configured to process the ISVD embeddings for an ISVD sequence and generate an output 109 that predicts a ISVD titer value for the corresponding ISVD sequence. The ISVD titer model 108 may be based on any suitable machine-learning algorithm or technique, and can include one or more of: a neural network, a K-nearest neighbors model, a support vector machine, a decision trees model, a random forest model, regression model (e.g., linear regression, XGBoost, and the like etc.), or a ridge regression model. The ISVD titer model 108 can be used to perform a classification task, or a regression task, and/or both. For example, the ISVD titer model 108 may be trained and configured to classify whether the titer of an ISVD sequence is a particular class label from a set of classes. For example, the ISVD titer model may be a binary classifier that outputs either a low titer class label or a high titer class label for each ISVD embedding of an ISVD sequence that is input. For example, a low titer class label is associated with titer values below a threshold titer value, and a high titer class label is associated with titer values greater than or equal to the threshold titer value. In another example, the ISVD titer model 108 may be a multi-class classifier that outputs, without limitation, for example a low titer class label, a middle titer class label, or a high titer class label for each ISVD embedding of an ISVD sequence that is input. For example, a low titer class label is associated with titer values below a first threshold titer value, a medium titer class label is associated with titer values between the first threshold titer value and a second threshold titer value, and a high titer class label is associated with titer values greater than or equal to the second threshold titer value. The ISVD titer model 108 may be an N-class classifier depending on the variability and statistic of the ISVD dataset used to train the ISVD titer model 108.

In other examples, the ISVD titer ML model 108 is a trained regression ML model configured for estimating a ISVD titer production value for each ISVD sequence of the ISVD sequence dataset based on the ISVD embeddings for said each ISVD sequence in the ISVD sequence dataset as input. Alternatively, the ISVD titer ML model 108 may be trained as both a classifier and regressor and output a class label and a predicted titer production value.

In some implementations, the system 100 or another system is configured to train the ISVD titer model 108 and/or update the ISVD titer model 108. In this example, the system 100 includes a machine learning engine 108b configured to train and/or update model parameters 108c associated with the ISVD titer model 108. The machine learning engine 108b may perform unsupervised and/or supervised learning. In this example, the machine learning engine 108b performs supervised learning using stored labelled ISVD training dataset(s) 108a. Given that the ISVD titer model 108 processes ISVD embeddings 106 of each ISVD sequence for outputting a predicted titer value 109, the labelled ISVD training dataset(s) 108a are in fact labelled ISVD embedding dataset(s). For example, a labelled ISVD training dataset 108a includes a plurality of labelled ISVD training data instances, in which each ISVD training data instance corresponds to the ISVD embeddings 106 of an ISVD sequence and labelled with a measured titer production value for that ISVD sequence. An ISVD sequence dataset including a plurality of unique ISVD sequences in which each ISVD sequence is labelled with a measured titer production value may be processed by the protein large language embeddings model 104 to generate the labelled training dataset 108a. Each ISVD sequence in the ISVD dataset is processed by the protein large language embeddings model 104 to generate corresponding ISVD embeddings for said each ISVD sequence. The ISVD embeddings for each ISVD sequence are stored as a training data instance and labelled or annotated with the measured titer production value for that ISVD sequence. So, given an ISVD dataset with measured production titer values, a labelled ISVD training dataset 108a may be generated and used to train the model parameters 108c of the ISVD titer model 108. The machine learning engine 108b is configured to perform supervised training of the model parameters 108c using a machine learning algorithm or technique to generate the ISVD titer model 108 using the labelled training dataset 108a. The labelled training dataset 108a may also be updated and used to update the model parameters 108c of an already trained ISVD titer model 108.

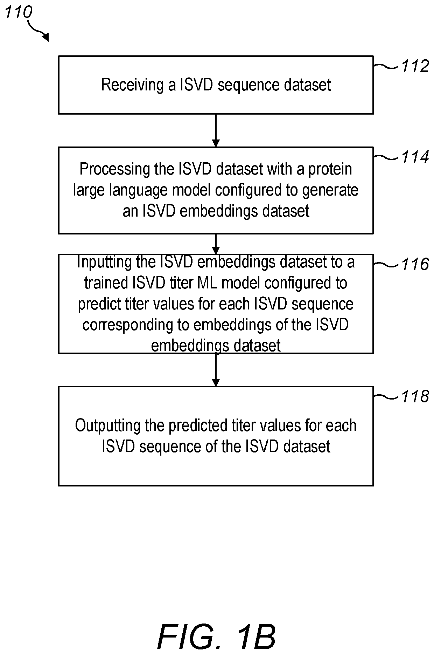

Figure 1b illustrates an example ISVD titer prediction process 110 for estimating titer of ISVDs in the system 100 of Figure 1a according to some embodiments of the invention. The example ISVD titer prediction process 110 includes at least the following steps of:

In step 112, receiving an ISVD sequence dataset including data representative of at least one ISVD sequence. The ISVD sequence dataset may include a plurality of ISVD sequences. Each ISVD sequence is a representation of the amino acid sequence of the ISVD.

In step 114, processing the received ISVD sequence dataset with a protein large language embeddings model configured for generating an ISVD embeddings dataset. The protein large language embeddings model may be a protein large language model trained and configured to output a sequence of tokens corresponding to each ISVD sequence in the ISVD dataset that is input, where each sequence of tokens is aggregated/averaged to form ISVD embeddings (a mean representation) corresponding to each ISVD sequence. The ISVD embeddings for a ISVD sequence may be represented as an embeddings vector of fixed length N. The ISVD sequence dataset is thus processed by the protein large language embedding model resulting in an output of the ISVD embeddings dataset. Alternatively, the protein large language embeddings model may be configured to output from a selected one or more hidden layers of the protein large language model the ISVD embeddings dataset. The ISVD embeddings dataset includes, for each ISVD sequence in the ISVD sequence dataset, a corresponding embeddings vector, wherein each element corresponds to a embedding.

In step 116, processing the ISVD embeddings dataset with a ML titer model configured for generating an estimated ISVD titer value for each ISVD sequence of the ISVD sequence dataset based on the corresponding ISVD embeddings of the ISVD embeddings dataset as input to the ML titer model.

In step 118, outputting data representative of the ISVD titer values corresponding to the ISVD sequence dataset for downstream processing.

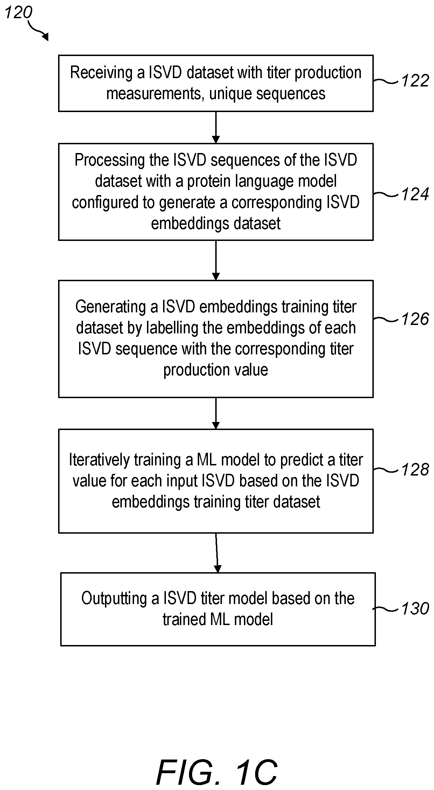

Figure 1c illustrates an example ISVD titer training process 120 for use in system 100 of Figure 1a according to some embodiments of the invention. The example ISVD titer training process 120 includes at least the following steps of:

In step 122, receiving an ISVD sequence dataset with associated titer production measurements or values for each ISVD sequence in the ISVD sequence dataset. The titer production measurements may be performed by any type of experimental titer measurement technique/process used to measure the titer of ISVD sequences. For example, the titer of an ISVD sequence can be determined using various laboratory methods, such as, without limitation, for example with spectrophotometry (A280), Bradford assay, BCA assay, ELISA, and SDS-PAGE/Western blot, or any other type of technique/methodology for measuring titer for ISVD sequences and the like.

In step 124, processing the ISVD sequence dataset with a protein large language model configured to generate ISVD embeddings for each ISVD sequence as described with reference to Figures 1a and 1b.

In step 126, generating an ISVD embeddings training titer dataset or ISVD training dataset 108a based on associating the ISVD embeddings for each ISVD sequence with the corresponding titer production measurement or value of said each ISVD sequence in the received ISVD dataset. The ISVD embeddings training titer dataset that is generated may be output and stored as the ISVD training dataset 108 in system 100 of Figure 1a. The ISVD training dataset 108 includes a plurality of ISVD training data instances, in which each ISVD training data instance included data representative of the ISVD embeddings for each ISVD sequence and the corresponding titer production measurement or value. For example, each ISVD training data instance includes data representative of an embeddings vector of fixed length *N* for a corresponding ISVD sequence and the corresponding titer production measurement or value.

In step 128, iteratively training an ML model to predict a titer value for each input ISVD based on using the ISVD embeddings training dataset as input.

In step 130, outputting a ISVD titer model based on the trained ML model.

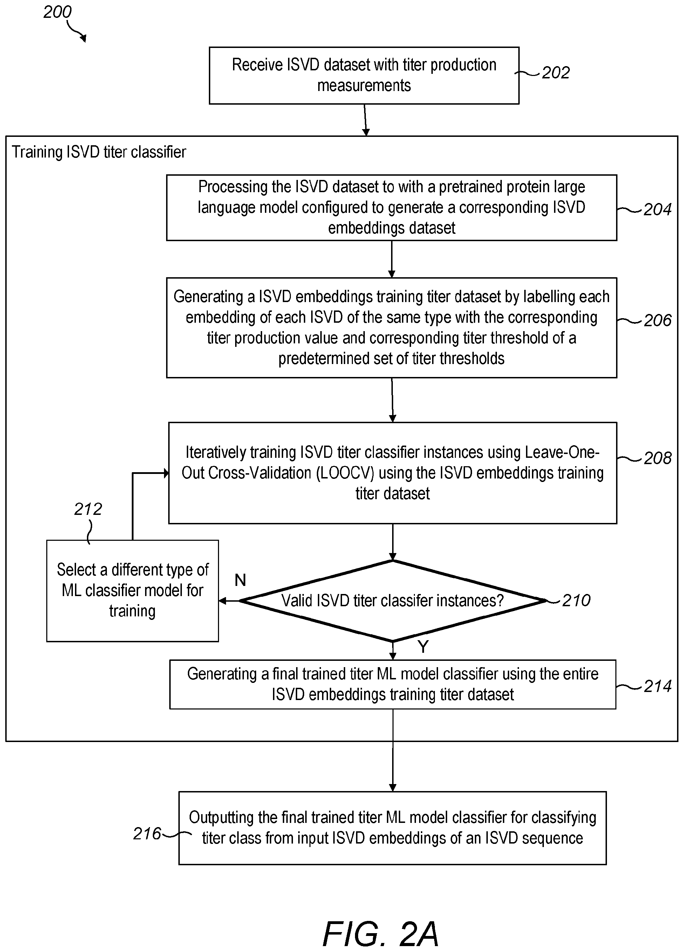

Figure 2a illustrates an example ISVD titer classifier training process 200 according to some embodiments of the invention. The example ISVD titer classifier training process 200 includes at least the following steps of:

In step 202, receiving an ISVD sequence dataset with associated titer production measurements or values for each ISVD sequence in the ISVD sequence dataset. The titer production measurements may be performed by any type of experimental titer measurement technique/process used to measure the titer of ISVD sequences. For example, the titer of an ISVD sequence can be determined using various laboratory methods, such as, without limitation, for example with spectrophotometry (A280), Bradford assay, BCA assay, ELISA, and SDS-PAGE/Western blot, or any other type of technique/methodology for measuring titer for ISVD sequences and the like.

In step 204, processing the ISVD sequence dataset with a pre-trained protein large language embeddings model configured to generate an ISVD embeddings dataset. The ISVD embedding dataset including ISVD embeddings (or protein-descriptors) for each ISVD sequence of the ISVD sequence dataset. For example, each of the ISVD embeddings for each ISVD sequence may form an embeddings vector of fixed length $N$. Each element of the embeddings vector being an embedding (e.g., protein-descriptor) associated with the ISVD sequence.

In step 206, generating an ISVD training titer dataset by labelling the ISVD embeddings for each ISVD sequence with the corresponding titer production measurement or value of said each ISVD sequence and corresponding titer thresholds of a predetermined set of titer thresholds corresponding to a set of classes for classification.

In step 208, iteratively training ISVD titer classifier instances using leave-one-out cross-validation (LOOCV) or other type of cross-validation technique using the ISVD training titer dataset and a particular ML classifier algorithm, where each ISVD titer classifier instance is trained to classify the ISVD embeddings for each ISVD sequence as a particular titer class of the set of titer classes using the predetermined set of titer thresholds and the titer production measurements of each of the training titer data instances.

As an example, once trained, each ISVD titer classifier instance may be configured for classifying each ISVD sequence of the ISVD sequence dataset with an ISVD titer class from a set of ISVD titer class labels when given the ISVD embedding dataset as input. The ISVD titer classifier processes the ISVD embedding dataset based on inputting the ISVD embeddings for each ISVD sequence to the ISVD titer classifier. The ISVD titer classifier processes the ISVD embeddings for each ISVD sequence and classifies said each ISVD sequence with an ISVD titer class from the set of ISVD class labels.

The set of ISVD titer class labels may include at least a low titer class label and a high titer class label. For example, the low titer class label corresponds to ISVD sequences classified as having a titer value less than a first threshold titer value, and the high titer class label corresponds to ISVD sequences classified as having a titer value greater than or equal to a second threshold titer value. The first threshold titer value is less than or equal to the second threshold titer value. For example, the ISVD titer classifier instance may be a binary classifier, where the first and second threshold titer values are the same. In another example, the ISVD titer classifier instance may be a multi-class classifier, and the set of ISVD titer class labels includes at least a low titer class label, a medium titer class label and a high titer class label, where the first and second threshold titer values are different, and the medium titer class label corresponds to ISVD sequences classified as having a titer production value between the first and second threshold titer values. This can be extended to additional titer classes using additional threshold titer values and corresponding titer class labels as the application demands.

In step 210, determining whether the ISVD titer classifier instances are validly trained. For example, the prediction performance of the ISVD titer classifier instances are greater than a desired prediction performance threshold. If it is determined that the ISVD titer classifier instances are validly trained (e.g. Y), then proceed to step 214, otherwise (e.g., N) proceed to step 212.

In step 212, select a different type of ML classifier algorithm/technique from a set of ML classifier algorithms for training an ML classifier model. For example, the ML classifier may be trained using at least one ML classifier algorithm from the group of: a Boosting ML classifier algorithm comprising one or more of: a AdaBoost classifier algorithm; a LPBoost classifier algorithm; a TotalBoost classifier algorithm; a BrownBoost classifier algorithm; a XGBoost classifier algorithm; a MadaBoost classifier algorithm; a LogiBoost classifier algorithm; and any other suitable Boosting ML classifier algorithm suitable for classifying each ISVD sequence of the ISVD sequence dataset with an ISVD titer class from the set of ISVD titer class labels based on the ISVD embeddings dataset as input; a decision-tree based classifier algorithms comprising one or more of: a Random Forest based classifier algorithm; a Extra Trees based classifier algorithm; any other suitable decision-tree based classifier algorithm suitable for classifying each ISVD sequence of the ISVD sequence dataset with an ISVD titer class from the set of ISVD titer class labels based on the ISVD embeddings dataset as input; a Naive Bayes, NB, based classifier algorithm comprising one or more of: a Gaussian NB based classifier algorithm; any other suitable NB based classifier algorithm suitable for classifying each ISVD sequence of the ISVD sequence dataset with an ISVD titer class from the set of ISVD titer class labels based on the ISVD embeddings dataset as input; an Artificial Neural Network, ANN, based classifier algorithm including one or more of: feed-forward NN based classifier algorithm; convolutional based classifier algorithm; a recurrent network based classifier algorithm; and any other suitable ANN based classifier algorithm suitable for classifying each ISVD sequence of the ISVD sequence dataset with an ISVD titer class from the set of ISVD titer class labels based on the ISVD embeddings dataset as input; a K-nearest Neighbour based classifier algorithm; a Logistic Regression based classifier algorithm; a Ridge regression based classifier algorithm; a support vector based classifier algorithm; a stochastic gradient descent, SGD, based classifier algorithm; any other suitable ML classifier algorithm suitable for classifying each ISVD sequence of the ISVD sequence dataset with an ISVD titer class from the set of ISVD titer class labels based on the ISVD embeddings dataset as input.

In step 214, generating a final trained titer ML model classifier using the entire ISVD training dataset and the ML classifier algorithm used to train the determined validly trained ISVD classifier instances.

As an option, even when it is determined that the trained ISVD titer classifier instances are valid, the process 200 may still proceed to step 212 for selecting a different type of ML classifier algorithm from a set of ML classifier algorithms for training another ML classifier model. Once the last ML classifier algorithm from the set of ML classifier algorithms has been used for training ISVD titer classifier instances, then the best performing of the validly trained ML classifier instances for each different type of ML classifier algorithm may be selected. The method 200 may proceed to step 214, where the final trained titer ML model classifier is trained using the selected ML classifier algorithm and the entire ISVD embeddings training titer dataset.

In step 216, outputting the final trained ISVD titer ML model classifier for classifying titer class when ISVD embeddings of an ISVD sequence are input to the final ISVD titer ML model classifier.

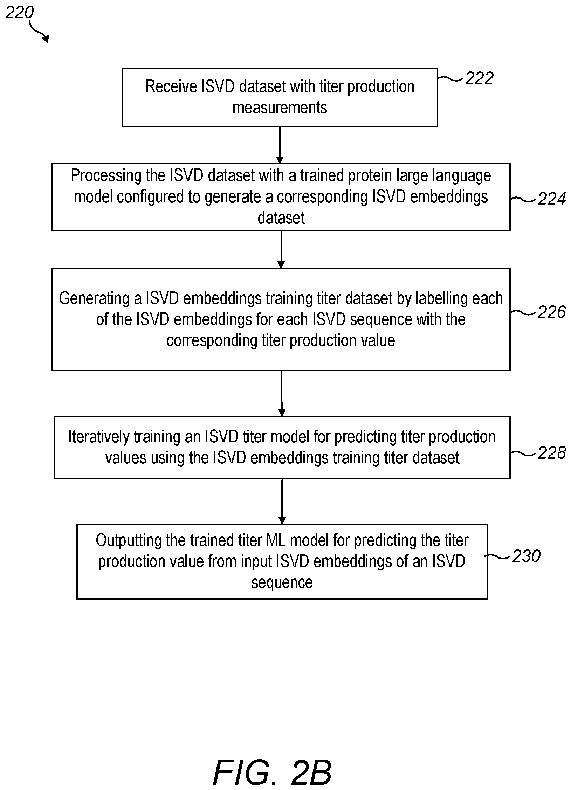

Figure 2b illustrates another example ISVD titer regressor training process 220 according to some embodiments of the invention. The example ISVD titer regressor training process 220 includes at least the following steps of:

In step 222, receiving an ISVD sequence dataset with associated titer production measurements or values for each ISVD sequence in the ISVD sequence dataset. The titer production measurements may be performed by any type of experimental titer measurement technique/process used to measure the titer of ISVD sequences. For example, the titer of an ISVD sequence can be determined using various laboratory methods, such as, without limitation, for example with spectrophotometry (A280), Bradford assay, BCA assay, ELISA, and SDS-PAGE/Western blot, or any other type of technique/methodology for measuring titer for ISVD sequences and the like.

In step 224, processing the ISVD sequence dataset with a pre-trained protein large language model configured to generate an ISVD embeddings dataset. The ISVD embeddings dataset including ISVD embeddings for each ISVD sequence of the ISVD sequence dataset.

In step 226, generating an ISVD training titer dataset by labelling the ISVD embeddings for each ISVD sequence with the corresponding titer production measurement or value of said each ISVD sequence.

In step 228, iteratively training ISVD titer model using a ML regression algorithm or technique, where the ISVD titer model is configured for predicting titer production values using the training titer dataset.

As an example, the ML regression algorithm or technique may include at least one from the group of: regression learning algorithm; neural network; extreme gradient boost regressor algorithm; Adaptive Boosting algorithm; bagging algorithms; Gradient boosting algorithm; any other statistical regression algorithm; any other ML regression algorithm suitable for training model parameters of an ML model for predicting the titer production value when given an ISVD embeddings of a ISVD sequence as input.

In step 230, outputting the trained ISVD titer ML regression model for predicting titer production values when ISVD embeddings of an ISVD sequence are input to the ISVD titer ML regression model.

The ISVD titer classifier model and/or ISVD titer regression model as described with reference to Figures 2a and 2b may be used for predicting titer values for ISVD sequences having an unknown titer. Thus, titer estimation of the ISVD sequences with unknown titer may be performed in silico using the above-mentioned ISVD titer classifier model and/or ISVD titer regression model. This means that the demanding titer measurement processes may only be performed on those ISVD sequences that are predicted to have a "high" titer or a desired titer according to the output of the ISVD titer classifier model and/or ISVD titer regression model. This alleviates the cost and complexity in performing titer measurements over an entire ISVD sequence dataset.

Additional downstream processing may also include generating a shortlist of ISVDs based on those ISVD sequences with unknown titer in the received ISVD sequence dataset with an estimated ISVD titer value meeting a specified titer criteria. The ISVD sequences with unknown titer are processed using the selected protein large language model and corresponding ISVD titer classifier model and/or ISVD titer regression model for generating predicted titer classes/values for each of the ISVD sequences with unknown titer. This may be used to generate a shortlist of ISVDs meeting a specified titer criteria. For example, the shortlist of ISVD molecules may correspond to ISVD sequences expressed in an in vitro wet lab analysis or assay (or culture) based on the interaction of one or more compounds or molecules resulting in expression of the ISVDs, and selecting the one or more compounds and/or ISVDs in the shortlist for further analysis associated with *in vivo* trials, *in vitro* wet lab analysis, and/or high-throughput screening laboratory analysis.

There are many methods or techniques for performing *in silico* generation of ISVD sequences which leverage bioinformatics, computational biology, and machine learning techniques such as, without limitation, for example, template-based design techniques, De Novo design techniques, simulation of natural variability techniques, in silico affinity maturation, optimisation for stability and expression techniques, design validation techniques, whole cell modelling techniques, and/or any other bioinformatic, computational biology and/or ML technique for generating in silico ISVD sequences, which may be estimated to have particular types of properties and the like. After one or more ISVD sequences have been created and/or optimised *in silico,* the corresponding titer values / classes for such *in silico* generated ISVD sequences may still be unknown or require independent validation. In such situations, the ISVD titer classifier model and/or ISVD titer regression model as described with reference to Figures 2a and 2b is applied to the generated ISVD sequences to predict or estimate titer classes/values for each *in silico* generated ISVD sequence with an unknown titer or with titer values that require independent validation. Those in silico ISVD sequences meeting a specific or desired titer criteria based on the titer classes/values output from the ISVD titer classifier model and/or ISVD titer regression model are selected and shortlisted for further downstream analysis. This provides the advantage of more rapid exploration and refinement of ISVD sequences during therapeutic ISVD/antibody development before experimental validation via, without limitation, for example *in vivo* trials, *in vitro* wet lab analysis, and/or high-throughput screening laboratory analysis and the like.

*In silico* generation of ISVD sequences can leverage bioinformatics, computational biology, and machine learning techniques to create and optimise ISVD sequences with desired properties, where the ISVD titer classifier model and/or ISVD titer regression model as described with reference to Figures 2a and 2b can be applied to predict or estimate titer classes/values for any generated ISVD sequences with an unknown titer. This may be used to generate a shortlist of ISVD sequences from the *in silica* generated ISVD sequences meeting a specified titer criteria. This can assist in downstream analysis such as therapeutic ISVD or antibody development, allowing for the rapid exploration and refinement of ISVD sequences before experimental validation via, without limitation, for example *in vivo* trials, *in vitro* wet lab analysis, and/or high-throughput screening laboratory analysis.

For example, ISVD sequences may be generated *in silica* based on, without limitation, for example *in silica* experiments using biological cell models/simulations configured for simulating or modelling the interaction of one or more compounds or molecules in a biological cell or culture resulting in expression of the ISVD sequences during the biological cell model/simulation. The simulation results may include any generated / expressed ISVD sequences. These ISVD sequences may be tested using the ISVD titer classifier model and/or ISVD titer regression model as described with reference to Figures 2a and 2b for predicting titer classes/values and whether such ISVD sequences meet specified titer criteria. Thus, these ISVD sequences and/or one or more compounds associated with such ISVD sequences meeting the specified titer criteria may be selected and shortlisted for further downstream analysis such as, without limitation, for example *in vivo* trials, *in vitro* wet lab analysis, and/or high-throughput screening laboratory analysis and the like.

Multivalent ISVD sequences may be generated *in silica* from monovalent building blocks. When generating such multivalent ISVD sequences *in silica* from a set of monovalent building blocks, the ordering of the selected monovalent building blocks from the set of monovalent building blocks can affect the properties of the ISVD sequences generated. Thus, a plurality of multivalent ISVD sequences may be generated from all ordering combinations of a particular set of monovalent building blocks. The ISVD titer classifier model and/or ISVD titer regression model as described with reference to Figures 2a and 2b can be used for predicting titer classes/values for the plurality of multivalent ISVD sequences. Each multivalent ISVD sequence from the plurality of multivalent ISVD sequences having a titer class/value that meet a specified titer criteria (e.g., titer class is a high titer class, or titer value is greater than a predetermined titer threshold) is selected and shortlisted for further downstream analysis. This may be used to determine the optimal ordering of the set of monovalent building blocks used to generate the selected multivalent ISVD sequence. The corresponding ordering from the set of monovalent building blocks used to generate each of the selected multivalent ISVD sequences may also be stored and/or linked to the corresponding selected multivalent ISVD sequence for further downstream analysis and/or for generating further ISVD sequences. This may be used to predict or estimate the optimal ordering combination of monovalent building blocks for generating multivalent ISVD sequences meeting a particular titer criteria.

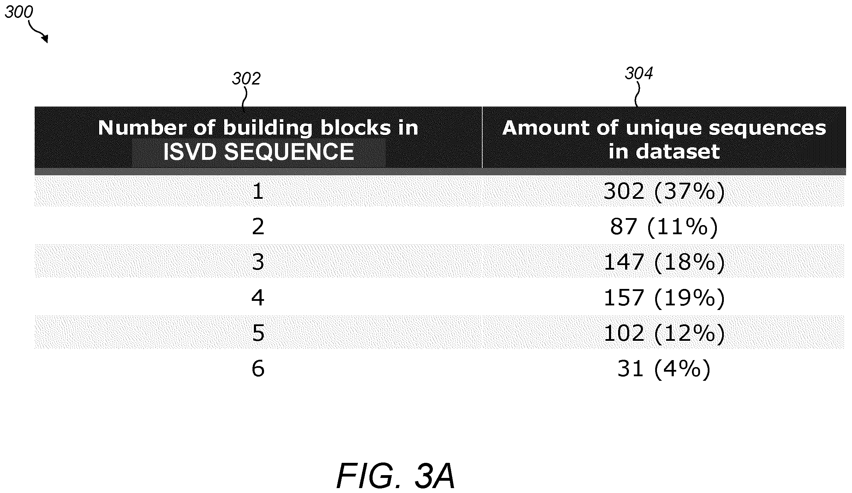

Figure 3a illustrates an example ISVD dataset 300 for use in the training ISVD model processes 100, 110, 200 of Figures 1a to 2a according to some embodiments of the invention. In this example, the initial ISVD dataset included 847 ISVD sequences with different numbers of monovalent and multivalent ISVDs (e.g., bivalent, trivalent, tetravalent, pentavalent and hexavalent) ranging from sequence lengths of 115 and 937 amino acids. Each different ISVD was measured for titer production from 1 to 44 times. The initial ISVD dataset was cleaned such as, for example, removing duplicate ISVD sequences where for any duplicate ISVD sequences the mean titer production value was computed and used as the titer production value for the corresponding ISVD sequence. The resulting ISVD dataset having unique ISVD sequences and corresponding titer production values was used as the ground truth for further *in silico* prediction modeling. The resulting titer production values of the resulting ISVD dataset range from 0.02g/L to 14.1g/L. In this example, binary classification was performed with a titer threshold of 2g/L being used to generate a low and high titer class, in which the low class for labelling ISVD sequences as low titer producers being those ISVD sequences with titer production values less than the titer threshold of 2g/L, or as high titer producers being those ISVD sequences with titer production values greater than the titer threshold of 2g/L for classification purposes. Although the titer threshold is described in this example as being 2g/L, this is by way of example only and the titer threshold is not so limited, it is to be appreciated by the skilled person that any suitable titer threshold may be used for classifying whether an ISVD sequence is low or high titer.

The resulting example ISVD dataset 300 is illustrated in Figure 3a with the first column 302 illustrates the different valency types with in the ISVD dataset 300 which range from monovalent (1), bivalent (2), trivalent (3), tetravalent (4), pentavalent (5) and hexavalent (6) ISVD sequences. The second column 304 illustrated the number of different or unique ISVD sequences within each valency type.

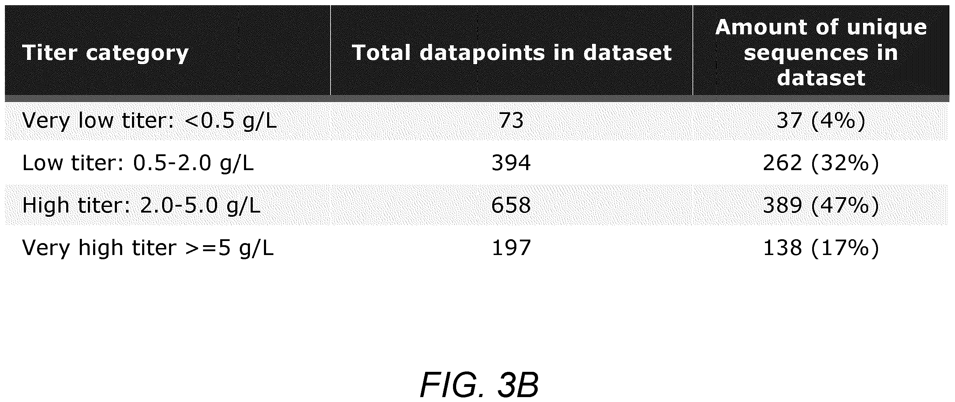

Figure 3b illustrates the example ISVD dataset 300 of Figure 3a for different ranges of titer thresholds according to some embodiments of the invention. The titer thresholds range from very low titer < 0.5 g/L, low titer >= 0.5 to < 2.0g/L, high titer >=2.0 to < 0.5 g/L, and very high titer >=5 g/L. This illustrates the spread of the ISVD sequences over various ranges of titer thresholds.

In this example, the titer threshold of 2g/L was used to generate a ISVD titer classifier model (binary classifier) using the training processes 110 and/or 200 as described with reference to Figures 1a to 2a. In this example, multiple ISVD titer classifier models were trained using different selected classifier algorithms, for each classifier algorithm for training the ISVD titer classifier model, a selected set of pre-trained protein large language models were used based on the ESM1 and ESM2 family of protein large language models (e.g., esm1v_t33_650M_UR905_1, esm2_t36_3B_UR50D). Each protein large language model combined with each classifier algorithm was assessed to determine the best pairing of protein large language model and classifier algorithm for generating the best ISVD titer model for each of the different valency types and a ISVD titer model for processing all valency types of ISVD sequences.

For each different pre-trained protein large language model and classifier algorithm pairing, an ISVD titer classifier model was generated for predicting the titer class of low or high, based on the experimental titer threshold of 2g/L as described above. In the examples, the classifiers used were from the python library scikit-learn (e.g., Pedregosa et al.,"Scikit-learn: Machine Learning in Python", The Journal of Machine Learning Research, Vol. 12, pages 2825-2830, 01 November 2011) in which each different classifier algorithm used 300 estimators with all other parameters being set-up as default.

As described with reference to Figure 2a, to ensure the validation of each ISVD titer classifier model, iterative training using leave-one-out cross-validation was performed, where through a loop, each unique ISVD sequence is used as testing group (e.g., n=1), while all other ISVD sequences in the dataset are used as training group (e.g., n=846). This ensures that in each iteration the resulting ISVD titer classifier model can use almost-all (n-1) ISVD sequences as training, while predicting blindly the left-out sequence (1) in that iteration of the loop. Once each ISVD titer classifier model is built by using all but one sequence (n-1), the left-out sequence is predicted blindly, and the outcome of the prediction (either high or low titer predicted production) is compiled, to then perform a statistical analysis of the performance of the ISVD titer classifier model by taking the whole blind predicted dataset. This was performed for each pre-trained protein large language model and classifier algorithm pairing resulting in a set of protein large language model and ISVD titer classifier model pairs, each of which were evaluated.

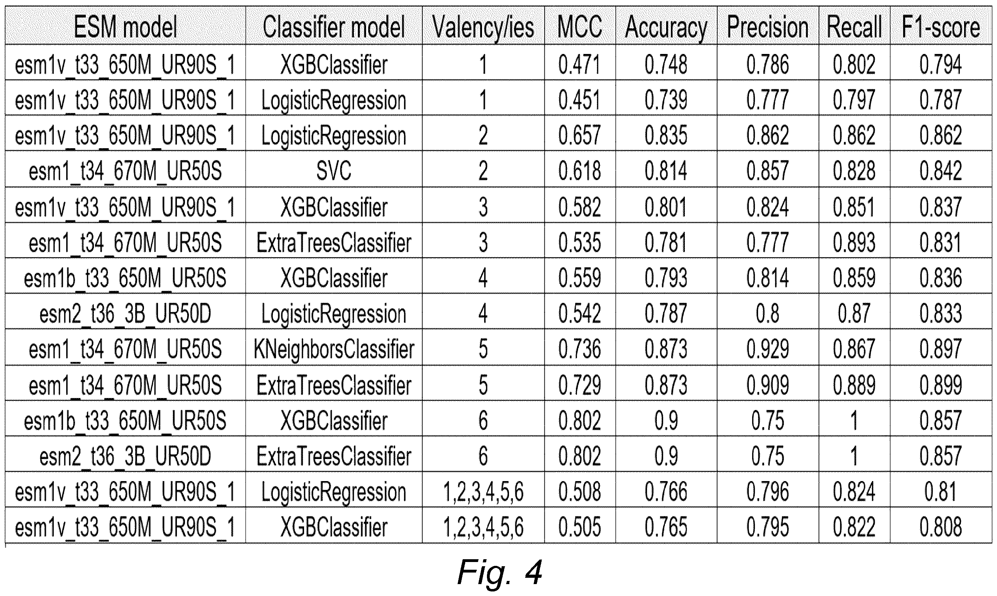

Figure 4 illustrates example titer classifier models trained for binary titer classification using the ISVD dataset of Figures 3a and 3b according to some embodiments of the invention. In this example, different combinations of protein large language models and ISVD titer ML classifier models were tested to determine the best performing protein large language model and ML classifier model combination for different types of ISVD valencies.

Initially, a set of different protein large language models were selected from the family of pre-trained ESM protein large language models. The set of ESM protein large language models that were selected for testing included, without limitation, for example esm1v_t33_650M_UR90S_1 (i.e., uses 650 million parameters/weights), esm1_t34_670M_UR50S 1 (i.e., uses 670 million parameters/weights), esm1b_t33_650M_UR50S 1 (i.e., uses 650 million parameters/weights), and esm2_t36_3B_UR50D 1 (e.g., uses 3 billion parameters/weights). Although various different ESM protein large language models are used, this for simplicity and by way of example only, it is to be appreciated by the skilled person that other types of protein large language models may be used and/or applied for generating embeddings from the original ISVD dataset with titer values for training corresponding ISVD titer ML classifier models and the like.

Each selected ESM protein large language model was used to generate, from the ISVD dataset of Figures 3a and 3b, a set of embedded ISVD training datasets. Each embedded ISVD training dataset for a particular ESM protein large language model corresponds to ISVD sequences having a different valency type or mixture thereof. In this example, each set of embedded ISVD training datasets generated by a particular ESM protein large language model included an embedded ISVD training dataset having monovalent ISVDs; an embedded ISVD training dataset having bivalent ISVDs; an embedded ISVD training dataset having trivalent ISVDs; an embedded ISVD training dataset having tetravalent ISVDs; an embedded ISVD training dataset having pentavalent ISVDs; and an embedded ISVD training dataset having hexavalent ISVDs; as well as an embedded ISVD training dataset including all ISVD valencies from the ISVD dataset of Figures 3a and 3b. In this manner, a plurality of sets of embedded ISVD training datasets was generated corresponding with the different selected ESM protein large language models and the different valency types.

In addition, a set of different ML classifier algorithms are selected for training corresponding ISVD titer ML classifier models based on the corresponding embedded ISVD training datasets output from the corresponding ESM protein large language models for respective ISVD valency values. Using the scikit-learn package, the set of ML classifier algorithms used to train corresponding ML classifier models included, without limitation, for example, XGBClassifier, RandomForestClassifier, SGDClassifier, LogisticRegression, SVC, ExtraTreesClassifier, KneighborsClassifier, RidgeClassifier, and GaussianNB. Although these different ML algorithms were tested, this is for simplicity and by way of example only, it is to be appreciated by the skilled person that other types of ML classifier algorithms may also be tested, used and/or applied for training, using corresponding embedded ISVD training datasets, corresponding ISVD titer ML classifier models and the like. It is noted that the default scikit learn package settings for each ML algorithm from the selected set of ML algorithms were used. Although the default scikit learn settings were applied, this is for simplicity and by way of example only, it is to be appreciated by the skilled person that other hyperparameters and/or parameter settings may be applied for training the corresponding ML algorithm for generating an ISVD titer ML classifier model.

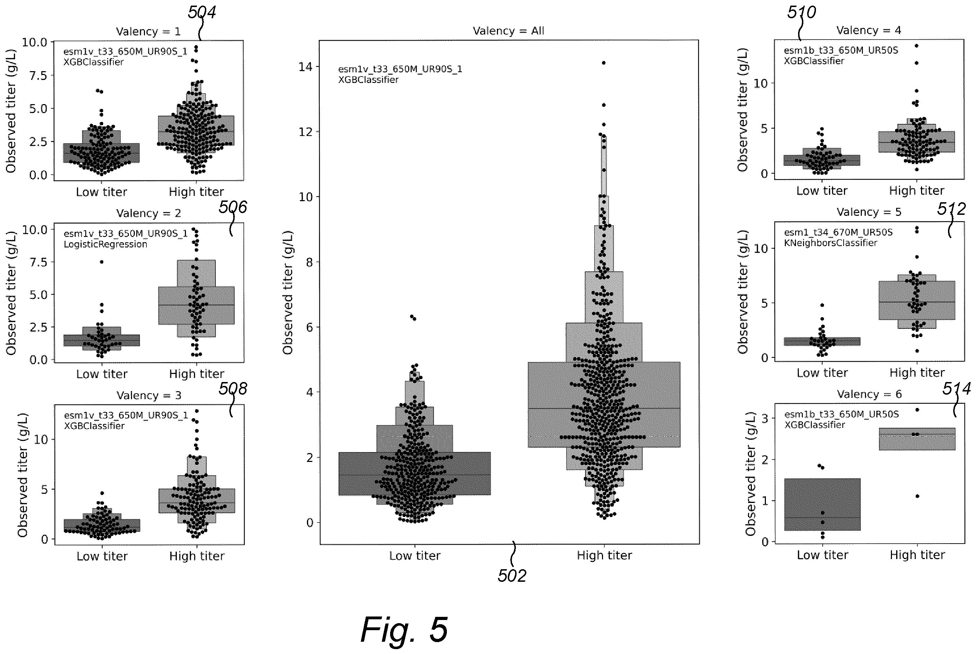

All combinations of the plurality of sets of embedded ISVD training datasets, generated from the set of protein large language models, and the ML algorithms from the set of ML classifier algorithms were used in training a corresponding plurality of ISVD titer ML classifier models. The performance metrics of each of the ISVD titer ML classifier models for each type of ISVD valency (e.g., a single valency value of 1, 2, 3, 4, 5, or 6, or all valency values from 1 to 6) was analysed with the two best performing combination of ESM protein language model and ISVD titer ML classifier model for each type of ISVD valency are illustrated in the performance table of Figure 4. The performance metrics analysed include, without limitation, for example the Matthews correlation coefficient (MCC) defining the correlation between the predicted and actual binary outcomes of a binary classifier, considering all four elements of a confusion matrix; Accuracy defined by the ratio of the number of correct predictions of the titer class with the total number of predictions; Precision defined by the ratio of the number of true positives with the total number of positives (e.g., true positives and false positives); Recall defined by the ratio of the number of true positives with the total number of true positives and false negatives; and F1-score defined by the harmonic mean of Precision and Recall. The performance statistics table of Figure 4 shows that titer class for unknown ISVD sequences having a valency from 1 to 6 can be reliably predicted from a particular pre-trained large language model and classifier model pairing. This is further illustrated in the performance plots 504-514 of Figure 5.

Figure 5 illustrates example performance plots 502-514 of boxenplot and swarmplot performance results for selected example trained ISVD titer classifier models of Figure 4 when predicting titer values for each different ISVD valency (e.g., Valencies 1 to 6).

Performance plot 502 illustrates the quality of the performance of the best pre-trained large language model and classifier model pairing for predicting titer over all valency types (e.g., Valency = {1, 2, 3, 4, 5, 6}). In this case, the protein large language model that was selected is the esm1v_t33_650M_UR90S_1 for generating embeddings of the ISVD dataset, and the ISVD titer classifier model is trained using the XGBClassifier. In this case, the performance of the ISVD titer classifier model when using the esm1v_t33_650M_UR90S_1 for generating the input ISVD embeddings is illustrated in the boxenplot and swarmplot illustrated in performance plot 502. It was found that the combination of the pre-trained protein large language model based on esm1v_t33_650M_UR905_1 for generating ISVD embeddings for each ISVD sequence and using a classifier algorithm based on XGBClassifier for training the ISVD titer classifier model from scikit-learn using the training process as described with reference to figures 1a to 2a resulted in an ISVD titer classifier model that was capable of predicting titer class (low titer, high titer) for ISVD sequences having different types of valencies. In this case, a statistical test was performed on the predicted versus observed mean titer predictions. The performance plot 502 illustrates a boxenplot of the distribution of the predicted class (x-axis) and the real observed mean titer (g/L) (y-axis). On top of the boxenplot, a swarmplot was drawn to represent each of the 847 unique mean titer determinations of the mono- and multivalent ISVDs. A statistical test was performed on the observed mean titer (g/L) values from the two different predicted class distributions (low and high titer) based on the Mann-Whitney-Wilcoxon two-sided with Bonferroni correction test. This yielded a p-value $\leq 1 \times 10^{-4}$ ($2.7 \times 10^{-41}$).

Each of the datapoints shown in the performance plot 502, are the outcome of a completely blind prediction (agnostic to the sequence that is being predicted), thereby, providing enough robustness to the resulting ISVD titer classifier model when it comes to predicting titer for new and unknown sequences. That is, if a new ISVD sequence is input and the titer class predicted, then the likelihood that the predictions are true is very high.

Performance plot 504 illustrates the quality of the performance of the best pre-trained large language model and classifier model pairing for predicting titer over for only monovalent ISVDs (Valency = 1). It was found that the combination of the pre-trained protein large language model based on esm1v_t33_650M_UR90S_1 for generating ISVD embeddings for each ISVD sequence and using a classifier algorithm based on XGBClassifier for training the ISVD titer classifier model from scikit-learn using the training process as described with reference to figures 1a to 4 resulted in an ISVD titer classifier model that was capable of predicting titer class (low titer, high titer) for only monovalent ISVD sequences. The performance of the resulting ISVD titer classifier model is illustrated in the boxenplot and swarmplot illustrated in performance plot 504. In this case, a statistical test was performed on the predicted versus observed mean titer predictions. The performance plot 504 illustrates a boxenplot of the distribution of the predicted class (x-axis) and the real observed mean titer (g/L) (y-axis). On top of the boxenplot, a swarmplot was drawn to represent from the 847 ISVD sequences with titer values those unique mean titer determinations that are monovalent ISVDs. The Mann-Whitney-Wilcoxon two-sided with Bonferroni correction test yielded a p-value $<<< 1 \times 10^{-4}$.

Each of the datapoints shown in the performance plot 504, are the outcome of a completely blind prediction (agnostic to the sequence that is being predicted), thereby, providing enough robustness to the resulting ISVD titer classifier model when it comes to predicting titer for new and unknown sequences for monovalent ISVDs. That is, if a new monovalent ISVD sequence is input to the pre-trained protein large language model based on esm1v_t33_650M_UR90S_1 and the resulting embedding is input to the resulting ISVD titer classifier model, then the likelihood that the predicted titer class is a true positive is again very high.

Performance plot 506 illustrates the quality of the performance of the best pre-trained large language model and classifier model pairing for predicting titer over for only bivalent ISVDs (Valency = 2). It was found that the combination of the pre-trained protein large language model based on esm1v_t33_650M_UR90S_1 for generating ISVD embeddings for each ISVD sequence and using a classifier algorithm based on LogisticsRegression for training the ISVD titer classifier model from scikit-learn using the training process as described with reference to figures 1a to 4 resulted in an ISVD titer classifier model that was capable of predicting titer class (low titer, high titer) for only bivalent ISVD sequences. The performance of the resulting ISVD titer classifier model is illustrated in the boxenplot and swarmplot illustrated in performance plot 506. In this case, a statistical test was performed on the predicted versus observed mean titer predictions. The performance plot 506 illustrates a boxenplot of the distribution of the predicted class (x-axis) and the real observed mean titer (g/L) (y-axis). On top of the boxenplot, a swarmplot was drawn to represent from the 847 ISVD sequences with titer values those unique mean titer determinations that are bivalent ISVDs. Again, the Mann-Whitney-Wilcoxon two-sided with Bonferroni correction test yielded a p-value <<< $1 \times 10^{-4}$.

Each of the datapoints shown in the performance plot 506, are the outcome of a completely blind prediction (agnostic to the sequence that is being predicted), thereby, providing enough robustness to the resulting ISVD titer classifier model when it comes to predicting titer for new and unknown sequences for bivalent ISVDs. That is, if a new bivalent ISVD sequence is input to the pre-trained protein large language model based on esm1v_t33_650M_UR90S_1 and the resulting embedding is input to the resulting ISVD titer classifier model, then the likelihood that the predicted titer class is a true positive is again very high.

Performance plot 508 illustrates the quality of the performance of the best pre-trained large language model and classifier model pairing for predicting titer over for only trivalent ISVDs (Valency = 3). It was found that the combination of the pre-trained protein large language model based on esm1v_t33_650M_UR90S_1 for generating ISVD embeddings for each ISVD sequence and using a classifier algorithm based on XGBClassifier for training the ISVD titer classifier model from scikit-learn using the training process as described with reference to figures 1a to 4 resulted in an ISVD titer classifier model that was capable of predicting titer class (low titer, high titer) for only trivalent ISVD sequences. The performance of the resulting ISVD titer classifier model is illustrated in the boxenplot and swarmplot illustrated in performance plot 508. In this case, a statistical test was performed on the predicted versus observed mean titer predictions. The performance plot 508 illustrates a boxenplot of the distribution of the predicted class (x-axis) and the real observed mean titer (g/L) (y-axis). On top of the boxenplot, a swarmplot was drawn to represent from the 847 ISVD sequences with titer values those unique mean titer determinations that are trivalent ISVDs. The Mann-Whitney-Wilcoxon two-sided with Bonferroni correction test also yielded a p-value <<< $1 \times 10^{-4}$.

Each of the datapoints shown in the performance plot 508, are the outcome of a completely blind prediction (agnostic to the sequence that is being predicted), thereby, providing enough robustness to the resulting ISVD titer classifier model when it comes to predicting titer for new and unknown sequences for trivalent ISVDs. That is, if a new trivalent ISVD sequence is input to the pre-trained protein large language model based on esm1v_t33_650M_UR90S_1 and the resulting embedding is input to the resulting ISVD titer classifier model, then the likelihood that the predicted titer class is a true positive is again very high.

Performance plot 510 illustrates the quality of the performance of the best pre-trained large language model and classifier model pairing for predicting titer over for only tetravalent ISVDs (Valency = 4). It was found that the combination of the pre-trained protein large language model based on esm1b_t33_650M_UR50S for generating ISVD embeddings for each ISVD sequence and using a classifier algorithm based on XGBClassifier for training the ISVD titer classifier model from scikit-learn using the training process as described with reference to figures 1a to 4 resulted in an ISVD titer classifier model that was capable of predicting titer class (low titer, high titer) for only tetravalent ISVD sequences. The performance of the resulting ISVD titer classifier model is illustrated in the boxenplot and swarmplot illustrated in performance plot 510. In this case, a statistical test was performed on the predicted versus observed mean titer predictions. The performance plot 510 illustrates a boxenplot of the distribution of the predicted class (x-axis) and the real observed mean titer (g/L) (y-axis). On top of the boxenplot, a swarmplot was drawn to represent from the 847 ISVD sequences with titer values those unique mean titer determinations that are tetravalent ISVDs. The Mann-Whitney-Wilcoxon two-sided with Bonferroni correction test yielded a p-value <<< $1 \times 10^{-4}$.

Each of the datapoints shown in the performance plot 510, are the outcome of a completely blind prediction (agnostic to the sequence that is being predicted), thereby, providing enough robustness to the resulting ISVD titer classifier model when it comes to predicting titer for new and unknown sequences for tetravalent ISVDs. That is, if a new tetravalent ISVD sequence is input to the pre-trained protein large language model based on esm1v_t33_650M_UR50S and the resulting embedding is input to the resulting ISVD titer classifier model, then the likelihood that the predicted titer class is a true positive is again very high.

Performance plot 512 illustrates the quality of the performance of the best pre-trained large language model and classifier model pairing for predicting titer over for only pentavalent ISVDs (Valency = 5). It was found that the combination of the pre-trained protein large language model based on esm1_t34_670M_UR50S for generating ISVD embeddings for each ISVD sequence and using a classifier algorithm based on KNeighborsClassifier for training the ISVD titer classifier model from scikit-learn using the training process as described with reference to figures 1a to 4 resulted in an ISVD titer classifier model that was capable of predicting titer class (low titer, high titer) for only pentavalent ISVD sequences. The performance of the resulting ISVD titer classifier model is illustrated in the boxenplot and swarmplot illustrated in performance plot 512. In this case, a statistical test was performed on the predicted versus observed mean titer predictions. The performance plot 512 illustrates a boxenplot of the distribution of the predicted class (x-axis) and the real observed mean titer (g/L) (y-axis). On top of the boxenplot, a swarmplot was drawn to represent from the 847 ISVD sequences with titer values those unique mean titer determinations that are pentavalent ISVDs. The Mann-Whitney-Wilcoxon two-sided with Bonferroni correction test yielded a p-value $<<< 1 \times 10^{-4}$.

Each of the datapoints shown in the performance plot 512, are the outcome of a completely blind prediction (agnostic to the sequence that is being predicted), thereby, providing enough robustness to the resulting ISVD titer classifier model when it comes to predicting titer for new and unknown sequences for pentavalent ISVDs. That is, if a new pentavalent ISVD sequence is input to the pre-trained protein large language model based on esm1_t34_670M_UR50S and the resulting embedding is input to the resulting ISVD titer classifier model, then the likelihood that the predicted titer class is a true positive is again very high.

Performance plot 514 illustrates the quality of the performance of the best pre-trained large language model and classifier model pairing for predicting titer over for only hexavalent ISVDs (Valency = 6). It was found that the combination of the pre-trained protein large language model based on esm1b_t33_650M_UR50S for generating ISVD embeddings for each ISVD sequence and using a classifier algorithm based on XGBClassifier for training the ISVD titer classifier model from scikit-learn using the training process as described with reference to figures 1a to 4 resulted in an ISVD titer classifier model that was capable of predicting titer class (low titer, high titer) for only hexavalent ISVD sequences. The performance of the resulting ISVD titer classifier model is illustrated in the boxenplot and swarmplot illustrated in performance plot 514. In this case, a statistical test was performed on the predicted versus observed mean titer predictions. The performance plot 514 illustrates a boxenplot of the distribution of the predicted class (x-axis) and the real observed mean titer (g/L) (y-axis). On top of the boxenplot, a swarmplot was drawn to represent from the 847 ISVD sequences with titer values those unique mean titer determinations that are hexavalent ISVDs. The Mann-Whitney-Wilcoxon two-sided with Bonferroni correction test yielded a p-value $<<< 1 \times 10^{-4}$.

Each of the datapoints shown in the performance plot 514, are the outcome of a completely blind prediction (agnostic to the sequence that is being predicted), thereby, providing enough robustness to the resulting ISVD titer classifier model when it comes to predicting titer for new and unknown sequences for hexavalent ISVDs. That is, if a new hexavalent ISVD sequence is input to the pre-trained protein large language model based on esm1b_t33_650M_UR50S and the resulting embedding is input to the resulting ISVD titer classifier model, then the likelihood that the predicted titer class is a true positive is again very high. Although ISVD sequences with Valencies 1, 2, 3, 4, 5, 6 (e.g., monovalent, bivalent, trivalent, tetravalent, pentavalent, hexavalent) were illustrated and described with reference to Figures 4 and 5, this is for simplicity and by way of example only, it is to be appreciated by the skilled person that there will likely exist a particular pre-trained large language model and classifier model pairing that results in reliable titer class prediction for any one or more V-Valent ISVD sequences, where V>0, and/or multiple different valency ISVD combinations, and/or for all valency ISVDs. Although the ISVD titer classifier model and inference/prediction/classification results thereof are described herein in relation to monovalent ISVDs up to hexavalent ISVDs, this is by way of example only and the ISVD titer model / classifier model is not so limited. It is to be appreciated by the skilled person that the techniques, methods, apparatus, and systems described herein for generating, training, and/or operating the ISVD titer model / classifier model are applicable, subject to using suitable ISVD labelled training datasets including the ISVD sequence with the requisite valencies, to higher multivalency ISVDs such as, without limitation, for example heptavalent ISVDs, octavalent ISVDs, nonavalent ISVDs, decavalent ISVD, and beyond, and/or any suitable higher multivalency ISVDs as the application demands.

Although various selected types of ML classifier algorithms were described from the scikit learn package, this was for simplicity and by way of example only, it is to be appreciated by the skilled person that any suitable type of ML classifier algorithm may be used to train a biclass or multiclass ISVD titer classifier model for predicting titer values as described herein such as, without limitation, for example at least one ML classifier algorithm from the group of: a Boosting ML classifier algorithm comprising one or more of: a AdaBoost classifier algorithm; a LPBoost classifier algorithm; a TotalBoost classifier algorithm; a BrownBoost classifier algorithm; a XGBoost classifier algorithm; a MadaBoost classifier algorithm; a LogiBoost classifier algorithm; and any other suitable Boosting ML classifier algorithm suitable for classifying each ISVD sequence of the ISVD sequence dataset with an ISVD titer class from the set of ISVD titer class labels based on the ISVD embeddings dataset as input; a decision-tree based classifier algorithms comprising one or more of: a Random Forest based classifier algorithm; a Extra Trees based classifier algorithm; any other suitable decision-tree based classifier algorithm suitable for classifying each ISVD sequence of the ISVD sequence dataset with an ISVD titer class from the set of ISVD titer class labels based on the ISVD embeddings dataset as input; a Naïve Bayes, NB, based classifier algorithm comprising one or more of: a Gaussian NB based classifier algorithm; any other suitable NB based classifier algorithm suitable for classifying each ISVD sequence of the ISVD sequence dataset with an ISVD titer class from the set of ISVD titer class labels based on the ISVD embeddings dataset as input; an Artificial Neural Network (ANN) based classifier algorithm including one or more of: feed-forward neural network (NN) based classifier algorithm; convolutional based classifier algorithm; a recurrent NN based classifier algorithm; a Graph Neural Network algorithm (GNN); and any other suitable ANN based classifier algorithm suitable for classifying each ISVD sequence of the ISVD sequence dataset with an ISVD titer class from the set of ISVD titer class labels based on the ISVD embeddings dataset as input; a K-nearest Neighbour based classifier algorithm; a Logistic Regression based classifier algorithm; a Ridge regression based classifier algorithm; a support vector based classifier algorithm; a stochastic gradient descent (SGD) based classifier algorithm; any other suitable ML classifier algorithm suitable for classifying each ISVD sequence of the ISVD sequence dataset with an ISVD titer class from the set of ISVD titer class labels based on the ISVD embeddings dataset as input.

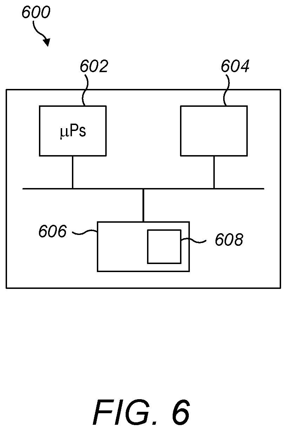

Figure 6 is a schematic illustration of a system/apparatus for performing methods described herein. The system/apparatus shown is an example of a computing device. It will be appreciated by the skilled person that other types of computing devices/systems may alternatively be used to implement the methods described herein, such as a distributed computing system.

The apparatus (or system) 600 comprises one or more processors 602. The one or more processors control operation of other components of the system/apparatus 600. The one or more processors 602 may, for example, comprise a general-purpose processor. The one or more processors 602 may be a single core device or a multiple core device. The one or more processors 602 may comprise a central processing unit (CPU) or a graphical processing unit (GPU). Alternatively, the one or more processors 602 may comprise specialised processing hardware, for instance a RISC processor or programmable hardware with embedded firmware. Multiple processors may be included.

The system/apparatus comprises a working or volatile memory 604. The one or more processors may access the volatile memory 604 in order to process data and may control the storage of data in memory. The volatile memory 604 may comprise RAM of any type, for example Static RAM (SRAM), Dynamic RAM (DRAM), or it may comprise Flash memory, such as an SD-Card.

The system/apparatus comprises a non-volatile memory 606. The non-volatile memory 606 stores a set of operation instructions 608 for controlling the operation of the processors 602 in the form of computer readable instructions or code. The non-volatile memory 606 may be a memory of any kind such as a Read Only Memory (ROM), a Flash memory, or a magnetic drive memory.

The one or more processors 602 are configured to execute operating instructions 608 to cause the system/apparatus to perform any of the methods or processes described herein with reference to Figures 1a to 5. The operating instructions 608 may also comprise instructions or code (i.e., drivers) relating to the hardware components of the system/apparatus 600, as well as instructions or code relating to the basic operation of the system/apparatus 600. Generally speaking, the one or more processors 602 execute one or more instructions or code of the operating instructions 608, which are stored permanently or semi-permanently in the non-volatile memory 606, using the volatile memory 604 to temporarily store data generated during execution of said operating instructions 608.

Implementations of the methods and/or processes described herein may be realised as in digital electronic circuitry, integrated circuitry, specially designed ASICs (application specific integrated circuits), computer hardware, firmware, software, and/or combinations thereof. These may include computer program products (such as software stored on e.g., magnetic discs, optical disks, memory, Programmable Logic Devices, in cloud storage, an app store and the like) comprising computer readable instructions or code that, when executed by a computer, such as that described in relation to Figure 6, cause the computer to perform one or more of the methods described herein.

Any system feature as described herein may also be provided as a method feature, and vice versa. As used herein, means plus function features may be expressed alternatively in terms of their corresponding structure. In particular, method aspects may be applied to system aspects, and vice versa.

Furthermore, any, some and/or all features in one aspect can be applied to any, some and/or all features in any other aspect, in any appropriate combination. It should also be appreciated that particular combinations of the various features described and defined in any aspects of the invention can be implemented and/or supplied and/or used independently.

Although several embodiments have been shown and described, it would be appreciated by those skilled in the art that changes may be made in these embodiments without departing from the principles of this disclosure, the scope of which is defined in the claims.

## Claims

1. A computer-implemented method for estimating a titer of immunoglobulin single variable domains, ISVDs, the method comprising:

   receiving an ISVD sequence dataset comprising data representative of at least one ISVD sequence;
   processing the received ISVD sequence dataset with a protein large language embeddings model configured for generating an ISVD embeddings dataset;

processing the ISVD embeddings dataset with a machine learning, ML, model configured for generating an estimated ISVD titer value for each ISVD sequence of the ISVD sequence dataset based on the ISVD embeddings dataset input; and

outputting data representative of the ISVD titer values corresponding to the ISVD sequence dataset for downstream processing.

2. The computer-implemented method as claimed in claim 1, wherein the ISVD sequence dataset comprises either:

a plurality of monovalent ISVDs with different amino acid sequence lengths;
a plurality of multivalent ISVDs with different amino acid sequence lengths; or
a plurality of different monovalent and multivalent ISVDs with different amino acid sequence lengths.

3. The computer-implemented method as claimed in claim 2, wherein the multivalent ISVDs comprise at least one or more from the group of:

bivalent ISVDs;
trivalent ISVDs;
tetravalent ISVDs;
pentavalent ISVDs;
hexavalent ISVDs;
$V$-valency ISVDs, where V>1.

4. The computer-implemented method as claimed in any preceding claim, wherein the ML model is an ML classifier configured for classifying each ISVD sequence of the ISVD sequence dataset with an ISVD titer class from a set of ISVD titer class labels based on the ISVD embeddings dataset as input, and said processing the ISVD embeddings dataset further comprising inputting the ISVD embeddings for each ISVD sequence to the ML classifier for classifying said each ISVD sequence with an ISVD titer class from the set of ISVD class labels, wherein the ISVD titer class is the generated estimated ISVD titer value.

5. The computer-implemented method as claimed in claim 4, wherein the set of ISVD titer class labels comprises at least a low titer class label and a high titer class label, wherein the low titer class label corresponds to ISVD sequences classified as having a titer value less than a first threshold titer value, and the high titer class label corresponds to ISVD sequences classified as having a titer value greater than or equal to a second threshold titer value, wherein the first threshold titer value is less than or equal to the second threshold titer value; and, optionally:

wherein the ML classifier is a binary classifier, and the first and second threshold titer values are the same; or wherein the ML classifier is a multi-class classifier, and the set of ISVD titer class labels comprises at least a low titer class label, a medium titer class label and a high titer class label, wherein the first and second threshold titer values are the different, and the medium titer class label corresponds to ISVD sequences classified as having a titer value between the first and second threshold titer values.

6. The computer-implemented method as claimed in any of claims 4 or 5, wherein the ML classifier is trained using at least one ML classifier algorithm from the group of:

a Boosting ML classifier algorithm;
a decision-tree based classifier algorithm;
a Naive Bayes, NB, based classifier algorithm;
an Artificial Neural Network, ANN, based classifier algorithm;
a K-nearest Neighbour based classifier algorithm;
a Logistic Regression based classifier algorithm;
a Ridge regression based classifier algorithm;
a support vector based classifier algorithm;
a stochastic gradient descent, SGD, based classifier algorithm;
any other suitable ML classifier algorithm suitable for classifying each ISVD sequence of the ISVD sequence dataset with an ISVD titer class from the set of ISVD titer class labels based on the ISVD embeddings dataset as input.

7. The computer-implemented method as claimed in any preceding claim, wherein the ML model is trained based on a training ISVD dataset comprising a plurality of training data instances, each training data instance comprising data representative of an ISVD sequence and a corresponding titer value associated with the ISVD sequence, and the computer-implemented method further comprising:
generating the training ISVD dataset by:

receiving a ISVD sequence dataset with associated titer values for each ISVD sequence in the ISVD sequence dataset;
processing the ISVD sequence dataset with the protein large language embeddings model to generate ISVD embeddings for each ISVD sequence;
associating the ISVD embeddings for each ISVD sequence with the corresponding titer value for said each ISVD sequence; and
outputting the training ISVD dataset, wherein each training ISVD data instance comprises the ISVD embeddings for each ISVD sequence and the corresponding titer value.

8. The computer-implemented method as claimed in claim 7, wherein when the ML model is to be trained as an ML classifier model, training said ML classifier model by:

annotating the training ISVD dataset based on labelling each ISVD training data instance with a class label from a set of class labels based on the corresponding titer value and the corresponding threshold titer values associated with each class label; and
training the ML classifier model based on the annotated training ISVD dataset.

9. The computer-implemented method of any preceding claim, wherein the downstream processing comprises generating a shortlist of ISVD sequences based on those ISVD sequences in the received ISVD sequence dataset with an estimated ISVD titer value that meet a specified titer criteria, and, optionally,
expressing the ISVD molecules on the shortlist *in vitro* based on the interaction of one or more compounds or molecules resulting in expression of the ISVD molecules, and selecting the one or more compounds and/or ISVD molecules in the shortlist for further analysis associated with *in vivo* trials, *in vitro* wet lab analysis, and/or high-throughput screening laboratory analysis.

10. The computer-implemented method of any preceding claim, further comprising:

generating a plurality of ISVD sequences *in silico* as the ISVD sequence dataset, wherein said protein large language embedding model and ML model processes the ISVD sequence dataset to predict or estimate an ISVD titer value for each of ISVD sequences in the ISVD sequence dataset;
generating a shortlist of ISVD sequences based on selecting ISVD sequences from the ISVD sequence dataset with predicted or estimated ISVD titer values meeting predetermined titer criteria; and
outputting the shortlist of ISVD sequences for ISVD sequence production of said ISVD sequences and subsequent *in vitro* analysis, assays, and/or high-throughput screening laboratory analysis.

11. The computer-implemented method of any preceding claim, wherein the ISVD embeddings for each ISVD sequence are in the form of a high dimensional vector of fixed length $N$, where $N \gg 0$ is the total number of ISVD embeddings used for representing each ISVD sequence.

12. The computer-implemented method of any preceding claim, wherein the protein large language embedding model comprises a protein large language model configured for outputting, for each ISVD sequence that is input, a sequence of $M$ embeddings vectors, each embeddings vector of fixed size $N$, and said protein large language embedding model is further configured for outputting a "mean" representations embeddings vector of fixed size $N$ as the ISVD embeddings for said each ISVD sequence based on computing an average of the corresponding sequence of $M$ embeddings vectors.

13. The computer-implemented method of claim 12, wherein the protein large language model is a pre-trained protein language model configured to receive a variable sized input vector representing each ISVD sequence and further configured to generate a fixed length output vector of length $N$, where $N \gg 0$, representing the ISVD embeddings.

14. An apparatus or computing system comprising a processor, a memory unit and a communication interface, wherein the processor is connected to the memory unit and the communication interface, wherein the processor and memory are configured to implement the computer-implemented method according to any of the preceding claims.

**15.** A computer program product comprising data or instruction code, which when executed on a processor, causes the processor to implement the computer-implemented method of any of claims 1 to 13.

FIG. 1A

110

112

Receiving a ISVD sequence dataset

114

Processing the ISVD dataset with a protein large language model configured to generate an ISVD embeddings dataset

116

Inputting the ISVD embeddings dataset to a trained ISVD titer ML model configured to predict titer values for each ISVD sequence corresponding to embeddings of the ISVD embeddings dataset

118

Outputting the predicted titer values for each ISVD sequence of the ISVD dataset

*FIG. 1B*

*120*

Receiving a ISVD dataset with titer production measurements, unique sequences —*122*

Processing the ISVD sequences of the ISVD dataset with a protein language model configured to generate a corresponding ISVD embeddings dataset —*124*

Generating a ISVD embeddings training titer dataset by labelling the embeddings of each ISVD sequence with the corresponding titer production value —*126*

Iteratively training a ML model to predict a titer value for each input ISVD based on the ISVD embeddings training titer dataset —*128*

Outputting a ISVD titer model based on the trained ML model —*130*

*FIG. 1C*

*200*

Receive ISVD dataset with titer production
measurements ⎯202

Training ISVD titer classifier

Processing the ISVD dataset to with a pretrained protein large
language model configured to generate a corresponding ISVD
embeddings dataset ⎯204

Generating a ISVD embeddings training titer dataset by labelling each
embedding of each ISVD of the same type with the corresponding
titer production value and corresponding titer threshold of a
predetermined set of titer thresholds ⎯206

Iteratively training ISVD titer classifier instances using Leave-One-
Out Cross-Validation (LOOCV) using the ISVD embeddings training
titer dataset ⎯208

*212*

Select a different type of
ML classifier model for
training

N ◄── Valid ISVD titer classifer instances? ⎯210

Y

Generating a final trained titer ML model classifier using the entire
ISVD embeddings training titer dataset ⎯214

Outputting the final trained titer ML model classifier for classifying
216⎯ titer class from input ISVD embeddings of an ISVD sequence

*FIG. 2A*

220

```
┌─────────────────────────────────────┐
│   Receive ISVD dataset with titer production  │──── 222
│             measurements              │
└─────────────────────────────────────┘
                  │
                  ▼
┌─────────────────────────────────────┐
│ Processing the ISVD dataset with a trained protein large language │
│ model configured to generate a corresponding ISVD embeddings │── 224
│                  dataset                 │
└─────────────────────────────────────┘
                  │
                  ▼
┌─────────────────────────────────────┐
│ Generating a ISVD embeddings training titer dataset by labelling each │
│ of the ISVD embeddings for each ISVD sequence with the │── 226
│          corresponding titer production value          │
└─────────────────────────────────────┘
                  │
                  ▼
┌─────────────────────────────────────┐
│ Iteratively training an ISVD titer model for predicting titer production │
│ values using the ISVD embeddings training titer dataset │── 228
└─────────────────────────────────────┘
                  │
                  ▼
┌─────────────────────────────────────┐
│ Outputting the trained titer ML model for predicting the titer │
│ production value from input ISVD embeddings of an ISVD │── 230
│                  sequence                 │
└─────────────────────────────────────┘
```

# FIG. 2B

300

| Number of building blocks in ISVD SEQUENCE | Amount of unique sequences in dataset |
|---|---|
| 1 | 302 (37%) |
| 2 | 87 (11%) |
| 3 | 147 (18%) |
| 4 | 157 (19%) |
| 5 | 102 (12%) |
| 6 | 31 (4%) |

*FIG. 3A*

EP 4 730 342 A1

| Titer category | Total datapoints in dataset | Amount of unique sequences in dataset |
|---|---|---|
| Very low titer: <0.5 g/L | 73 | 37 (4%) |
| Low titer: 0.5-2.0 g/L | 394 | 262 (32%) |
| High titer: 2.0-5.0 g/L | 658 | 389 (47%) |
| Very high titer >=5 g/L | 197 | 138 (17%) |

*FIG. 3B*

EP 4 730 342 A1

| ESM model | Classifier model | Valency/ies | MCC | Accuracy | Precision | Recall | F1-score |
|---|---|---|---|---|---|---|---|
| esm1v_t33_650M_UR90S_1 | XGBClassifier | 1 | 0.471 | 0.748 | 0.786 | 0.802 | 0.794 |
| esm1v_t33_650M_UR90S_1 | LogisticRegression | 1 | 0.451 | 0.739 | 0.777 | 0.797 | 0.787 |
| esm1v_t33_650M_UR90S_1 | LogisticRegression | 2 | 0.657 | 0.835 | 0.862 | 0.862 | 0.862 |
| esm1_t34_670M_UR50S | SVC | 2 | 0.618 | 0.814 | 0.857 | 0.828 | 0.842 |
| esm1v_t33_650M_UR90S_1 | XGBClassifier | 3 | 0.582 | 0.801 | 0.824 | 0.851 | 0.837 |
| esm1_t34_670M_UR50S | ExtraTreesClassifier | 3 | 0.535 | 0.781 | 0.777 | 0.893 | 0.831 |
| esm1b_t33_650M_UR50S | XGBClassifier | 4 | 0.559 | 0.793 | 0.814 | 0.859 | 0.836 |
| esm2_t36_3B_UR50D | LogisticRegression | 4 | 0.542 | 0.787 | 0.8 | 0.87 | 0.833 |
| esm1_t34_670M_UR50S | KNeighborsClassifier | 5 | 0.736 | 0.873 | 0.929 | 0.867 | 0.897 |
| esm1_t34_670M_UR50S | ExtraTreesClassifier | 5 | 0.729 | 0.873 | 0.909 | 0.889 | 0.899 |
| esm1b_t33_650M_UR50S | XGBClassifier | 6 | 0.802 | 0.9 | 0.75 | 1 | 0.857 |
| esm2_t36_3B_UR50D | ExtraTreesClassifier | 6 | 0.802 | 0.9 | 0.75 | 1 | 0.857 |
| esm1v_t33_650M_UR90S_1 | LogisticRegression | 1,2,3,4,5,6 | 0.508 | 0.766 | 0.796 | 0.824 | 0.81 |
| esm1v_t33_650M_UR90S_1 | XGBClassifier | 1,2,3,4,5,6 | 0.505 | 0.765 | 0.795 | 0.822 | 0.808 |

*Fig. 4*

EP 4 730 342 A1

Fig. 5

FIG. 6

## EUROPEAN SEARCH REPORT

**Application Number**

EP 24 30 6746

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2015/005475 A1 (KUCIA JUSTYNA [GB] ET AL) 1 January 2015 (2015-01-01) * paragraphs [0008], [0015], [0106], [0113]; figures 3A-3F * | 1-15 | INV. G16B40/20 |
| A | WO 2024/040031 A1 (GENENTECH INC [US]) 22 February 2024 (2024-02-22) * paragraphs [0003], [0004], [0006], [0028], [0050], [0052], [0066], [0138] * | 1-15 | |
| A | US 2008/187966 A1 (SIMMONS LAURA [US]) 7 August 2008 (2008-08-07) * paragraph [0290] * | 1-15 | |
| A | PACKIAM KULANDAI AROCKIA ET AL: "PERISCOPE-Opt: Machine learning-based prediction of optimal fermentation conditions and yields of recombinant periplasmic protein expressed in Escherichia coli", COMPUTATIONAL AND STRUCTURAL BIOTECHNOLOGY JOURNAL, vol. 20, 1 January 2022 (2022-01-01), pages 2909-2920, XP093258811, Sweden ISSN: 2001-0370, DOI: 10.1016/j.csbj.2022.06.006 * Abstract; chapter "2. Methods"; chapter "3. Results" * | 1-15 | TECHNICAL FIELDS SEARCHED (IPC) G16B G06N |

The present search report has been drawn up for all claims

| Place of search | Date of completion of the search | Examiner |
|---|---|---|
| Munich | 14 March 2025 | Schmidt, Karsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 30 6746

This annex lists the patent family members relating to the patent documents cited in the above-mentioned European search report.
The members are as contained in the European Patent Office EDP file on
The European Patent Office is in no way liable for these particulars which are merely given for the purpose of information.

14-03-2025

| Patent document cited in search report | | Publication date | Patent family member(s) | | Publication date |
|---|---|---|---|---|---|
| US 2015005475 A1 | | 01-01-2015 | AU | 2013214172 A1 | 14-08-2014 |
| | | | CA | 2863564 A1 | 08-08-2013 |
| | | | EP | 2855522 A1 | 08-04-2015 |
| | | | HK | 1209133 A1 | 24-03-2016 |
| | | | JP | 2015510397 A | 09-04-2015 |
| | | | US | 2015005475 A1 | 01-01-2015 |
| | | | WO | 2013113898 A1 | 08-08-2013 |
| WO 2024040031 A1 | | 22-02-2024 | NONE | | |
| US 2008187966 A1 | | 07-08-2008 | AU | 2004205684 A1 | 05-08-2004 |
| | | | CA | 2513113 A1 | 05-08-2004 |
| | | | EP | 1585768 A2 | 19-10-2005 |
| | | | US | 2004229310 A1 | 18-11-2004 |
| | | | US | 2008187966 A1 | 07-08-2008 |
| | | | WO | 2004065417 A2 | 05-08-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004068820 A
- WO 2006030220 A
- WO 2006003388 A
- WO 200518629 A
- WO 199404678 A
- WO 199634103 A
- WO 199923221 A
- WO 2008020079 A
- WO 2015173325 A

### Non-patent literature cited in the description

- **HAMERS-CASTERMAN et al.** *Nature*, 1993, vol. 363, 446-448
- **MUYLDERMANS**. *Reviews in Molecular Biotechnology*, 2001, vol. 74, 277-302
- **WARD et al.** *Nature*, 1989, vol. 341, 544
- **HOLT et al.** *Trends Biotechnol.*, 2003, vol. 21, 484
- **DAVIS ; RIECHMANN**. *Febs Lett.*, 1994, vol. 339, 285-290
- *Prot. Eng.*, 1996, vol. 9, 531-537
- **CONRATH et al.** *J. Biol. Chem.*, 2001, vol. 276 (10), 7346-7350
- **DAVIES ; RIECHMAN**. *FEBS*, 1994, vol. 339, 285
- *Biotechnol.*, 1995, vol. 13, 475
- *Prot. Eng.*, 1996, vol. 9, 531
- **RIECHMAN**. *J. Immunol. Methods*, 1999, vol. 231, 25
- **KINGMA et al.** Auto-encoding variational Bayes,. *arXiv: 1312.6114*, 2013
- **BROWN et al.** Language Models are Few-Shot Learners,. *Advances in Neural Information Processing Systems*, 2020, vol. 33, 1877-1901
- **DEVLIN et al.** BERT: Pre-training of Deep Bidirectional Transformers for Language Understanding,. *arXiv:1810.04805*, 2018
- **PEDREGOSA et al.** Scikit-learn: Machine Learning in Python. *The Journal of Machine Learning Research*, 01 November 2011, vol. 12, 2825-2830